# EUROPEAN PATENT APPLICATION

(11) **EP 4 455 147 A1**
(43) Date of publication of application: **30.10.2024**
(21) Application number: 22910205.8
(22) Date of filing: 23.12.2022
(51) Int. Cl.: C07D 487/22, A61K 31/4985, A61P 35/00

(54) **POLYMORPH OF KRAS INHIBITOR, PREPARATION METHOD THEREFOR, AND USE THEREOF**

(30) Priority: 24.12.2021 CN 202111607822; 12.12.2022 CN 202211609907
(71) Applicant: Genfleet Therapeutics (Shanghai) Inc., Shanghai 201203 (CN); Zhejiang Genfleet Therapeutics Co., Ltd., Shaoxing Binhai New City Shaoxing, Zhejiang 312000 (CN)
(72) Inventor: ZHOU, Fusheng, Shanghai 201203 (CN); ZHAO, Jinzhu, Shanghai 201203 (CN); CAO, Yudong, Shanghai 201203 (CN); XIONG, Lun, Shanghai 201203 (CN); SHI, Rongzhen, Shanghai 201203 (CN); LAN, Jiong, Shanghai 201203 (CN)
(74) Representative: Elzaburu S.L.P.
(86) International application number: PCT/CN2022/141476
(87) International publication number: WO 2023/116895

(57) **Abstract**

Provided are a polymorph of a KRAS inhibitor, a preparation method therefor, and a use thereof. Specifically disclosed is a pharmaceutically acceptable salt of a compound 1 or a polymorph of the compound or pharmaceutically acceptable salt thereof, and further disclosed are a preparation method for the salt or polymorph and an application of the salt or polymorph in the treatment and/or prevention of KRAS G12C mutation-related diseases.

## Description

### TECHNICAL FIELD

The present invention relates to the field of pharmaceuticals, and particularly, to a KRAS inhibitor polymorph, a method for preparing same, and use thereof.

### BACKGROUND

Lung cancer is the most prevalent cancer worldwide. In China, lung cancer ranks first among all cancers in morbidity, and possesses the highest morbidity and mortality in China. According to the data published by the American Cancer Society in 2016, about 1.8 million people develop lung cancer annually worldwide, of which nearly 80% are non-small cell lung cancers (NSCLCs). Among lung cancers, RAS gene mutations are identified in about 32% of lung cancers, and the mutation in any one of the three major subtypes of RAS gene (HRAS, NRAS, or KRAS) may lead to the development of tumors in humans. It has been reported that the highest mutation frequency among RAS genes was observed in the KRAS gene, and KRAS mutation was detected in 25-30% of tumors. By contrary, the rates of oncogenic mutations in NRAS and HRAS family members are much lower (8% and 3%, respectively). The most common KRAS mutations are found at residues G12 and G13 in the P-loop and residue Q61. The G12C mutation is a frequent mutation in the KRAS gene (glycine-12 mutation to cysteine). This mutation has been found in about 13% of cancers, about 43% of lung cancers, and almost 100% of MYH-related polyposis (familial colon cancer syndrome). Therefore, the development of an inhibitor for selectively inhibiting KRAS mutation is a prospective orientation. In order to improve the inhibitory activity against KRAS mutations and reduce the inhibitory activity against wild-type KRAS, the development of novel selective inhibitors against KRAS mutants with higher activity, better selectivity, and lower toxicity has great significance.

### SUMMARY

The present invention is intended to provide a pharmaceutically acceptable salt of compound 1 or a series of stable polymorphs of the compound or the pharmaceutically acceptable salt thereof, and a method for preparing same, and use thereof.

In a first aspect, the present invention provides a pharmaceutically acceptable salt of a compound, wherein the compound is compound 1 with a structure of

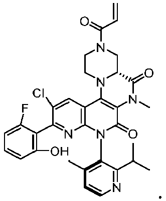

In another preferred embodiment, the pharmaceutically acceptable salt is a maleate, fumarate, citrate, p-toluenesulfonate, methanesulfonate, hydrochloride, or phosphate.

In another preferred embodiment, when the pharmaceutically acceptable salt is a maleate, fumarate, citrate, p-toluenesulfonate, or methanesulfonate, the pharmaceutically acceptable salt of the compound is an amorphous form.

In another preferred embodiment, in the maleate, the mole ratio of the compound to maleic acid is 1:1.

In another preferred embodiment, in the fumarate, the mole ratio of the compound to fumaric acid is 1:1.

In another preferred embodiment, in the citrate, the mole ratio of the compound to citric acid is 1:1.5.

In another preferred embodiment, in the p-toluenesulfonate, the mole ratio of the compound to *p-*toluenesulfonic acid is 1:1.

In another preferred embodiment, in the methanesulfonate, the mole ratio of the compound to methanesulfonic acid is 1:1.

In another preferred embodiment, when the pharmaceutically acceptable salt is a hydrochloride or phosphate, the pharmaceutically acceptable salt of the compound is a polymorph.

In another preferred embodiment, in the hydrochloride, the mole ratio of the compound to hydrochloric acid is 1:(0.9-1.1), e.g., 1:1.

In another preferred embodiment, in the phosphate, the mole ratio of the compound to phosphoric acid is 1:2.

In a second aspect, the present invention provides a polymorph, wherein the polymorph is a polymorph of a compound or a pharmaceutically acceptable salt of the compound; the compound is compound 1 with a structure of

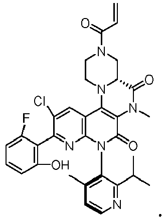

In another preferred embodiment, the pharmaceutically acceptable salt is a hydrochloride or phosphate.

In another preferred embodiment, the polymorph is crystalline form 1 of compound 1 hydrochloride having a characteristic X-ray powder diffraction peak at a 2• value selected from: 9.98±0.2°, 12.99±0.2°, 13.73±0.2°, 18.65±0.2°, 20.73±0.2°, 21.75±0.2°, 23.57±0.2°, 25.22±0.2°, and 27.27±0.2°.

In another preferred embodiment, the polymorph is crystalline form 1 of compound 1 hydrochloride, wherein the mole ratio of compound 1 to hydrochloric acid is 1:(0.9-1.1), e.g., 1:1. In another preferred embodiment, crystalline form 1 has a characteristic X-ray powder diffraction peak at a 2• value selected from: 8.37±0.2°, 9.98±0.2°, 11.69±0.2°, 12.99±0.2°, 13.73±0.2°, 15.08±0.2°, 18.65±0.2°, 19.87±0.2°, 20.73±0.2°, 21.58±0.2°, 21.75±0.2°, 23.57±0.2°, 25.22±0.2°, and 27.27±0.2°.

In another preferred embodiment, crystalline form 1 has a characteristic X-ray powder diffraction peak at a 2• value selected from: 8.37±0.2°, 9.98±0.2°, 10.91±0.2°, 11.69±0.2°, 12.99±0.2°, 13.73±0.2°, 15.08±0.2°, 16.07±0.2°, 18.40±0.2°, 18.65±0.2°, 19.87±0.2°, 20.73±0.2°, 21.58±0.2°, 21.75±0.2°, 23.57±0.2°, 25.22±0.2°, 26.79±0.2°, 27.27±0.2°, 27.61±0.2°, 29.07±0.2°, and 30.34±0.2°.

In another preferred embodiment, crystalline form 1 has characteristic X-ray powder diffraction peaks at one or more (e.g., 2, 3, 4, 5, 6, 7, 8, 9 or more or all) 2• values selected from Table 1.

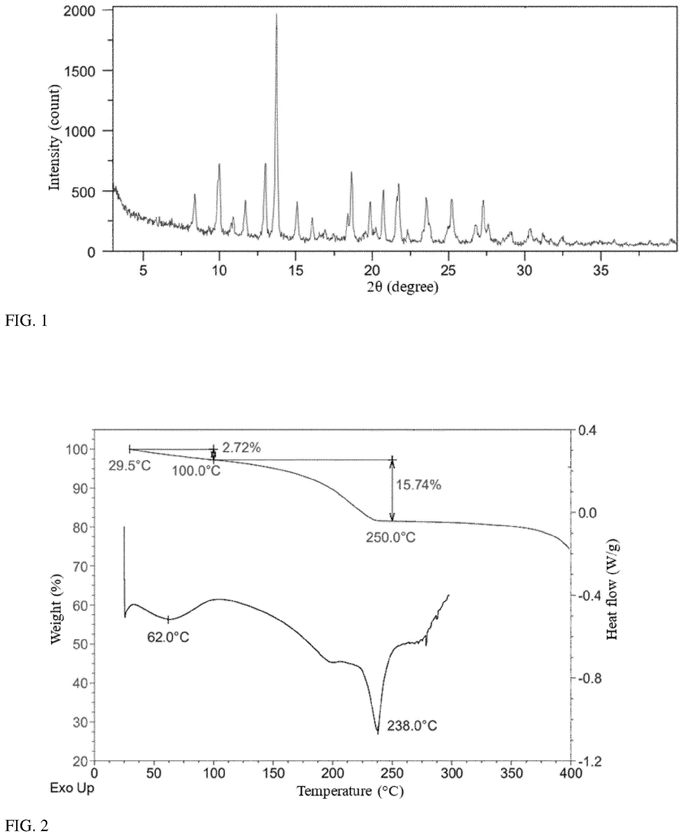

In another preferred embodiment, crystalline form 1 has an X-ray powder diffraction pattern substantially shown in FIG. 1.

In another preferred embodiment, crystalline form 1 has a differential scanning calorimetry pattern with characteristic peaks at 62.0±5 °C and 238.0±5 °C (endothermic peaks).

In another preferred embodiment, crystalline form 1 has a differential scanning calorimetry pattern with characteristic peaks at 62.0±1 °C and 238.0±1 °C (endothermic peaks).

In another preferred embodiment, crystalline form 1 has a differential scanning calorimetry pattern substantially shown in FIG. 2.

In another preferred embodiment, crystalline form 1 has a thermogravimetric analysis pattern with a 2.72%±0.5% weight loss at 100±5 °C (the weight loss percentage is the proportion of the weight loss to the weight of the sample before the weight loss) and a 15.7%±1% weight loss at 100±5 °C to 250±5 °C (the weight loss percentage is the proportion of the weight loss to the weight of the sample before the weight loss).

In another preferred embodiment, crystalline form 1 has a thermogravimetric analysis pattern with a 2.72%±0.2% weight loss at 100±1 °C (the weight loss percentage is the proportion of the weight loss to the weight of the sample before the weight loss) and a 15.7%±0.5% weight loss at 100±1 °C to 250±1 °C (the weight loss percentage is the proportion of the weight loss to the weight of the sample before the weight loss).

In another preferred embodiment, crystalline form 1 has a thermogravimetric analysis pattern substantially shown in FIG. 2.

In another preferred embodiment, the polymorph is crystalline form 2 of compound 1 phosphate having a characteristic X-ray powder diffraction peak at a 2• value selected from: 9.74±0.2°, 10.94±0.2°, 11.55±0.2°, 13.24±0.2°, 14.20±0.2°, 15.56±0.2°, 18.40±0.2°, 19.14±0.2°, 20.54±0.2°, 24.23±0.2°, and 25.88±0.2°.

In another preferred embodiment, the polymorph is crystalline form 2 of compound 1 phosphate, wherein the mole ratio of compound 1 to phosphoric acid is 1:2.

In another preferred embodiment, crystalline form 2 has a characteristic X-ray powder diffraction peak at a 2• value selected from: 9.74±0.2°, 10.94±0.2°, 11.55±0.2°, 13.24±0.2°, 14.20±0.2°, 15.56±0.2°, 16.02±0.2°, 16.91±0.2°, 18.40±0.2°, 19.14±0.2°, 19.53±0.2°, 20.54±0.2°, 24.23±0.2°, 24.67±0.2°, and 25.88±0.2°.

In another preferred embodiment, crystalline form 2 has a characteristic X-ray powder diffraction peak at a 2• value selected from: 7.37±0.2°, 9.74±0.2°, 10.94±0.2°, 11.55±0.2°, 13.24±0.2°, 14.20±0.2°, 15.56±0.2°, 16.02±0.2°, 16.91±0.2°, 18.40±0.2°, 19.14±0.2°, 19.53±0.2°, 20.54±0.2°, 21.12±0.2°, 22.35±0.2°, 24.23±0.2°, 24.67±0.2°, 25.88±0.2°, 27.44±0.2°, and 29.46±0.2°.

In another preferred embodiment, crystalline form 2 has characteristic X-ray powder diffraction peaks at one or more (e.g., 2, 3, 4, 5, 6, 7, 8, 9 or more or all) 2• values selected from Table 3.

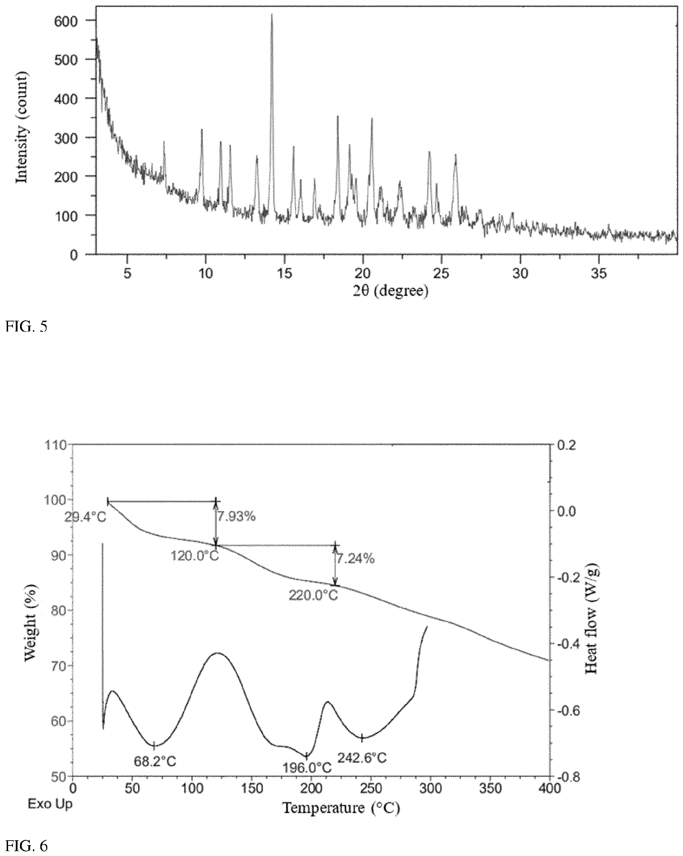

In another preferred embodiment, crystalline form 2 has an X-ray powder diffraction pattern substantially shown in FIG. 5.

In another preferred embodiment, crystalline form 2 has a differential scanning calorimetry pattern with characteristic peaks at 68.2±5 °C, 196.0±5 °C, and 242.6±5 °C (endothermic peaks).

In another preferred embodiment, crystalline form 2 has a differential scanning calorimetry pattern with characteristic peaks at 68.2±1 °C, 196.0±1 °C, and 242.6±1 °C (endothermic peaks).

In another preferred embodiment, crystalline form 2 has a differential scanning calorimetry pattern substantially shown in FIG. 6.

In another preferred embodiment, crystalline form 2 has a thermogravimetric analysis pattern with a 7.93%±1% weight loss at 120±5 °C (the weight loss percentage is the proportion of the weight loss to the weight of the sample before the weight loss) and a 7.24%±1% weight loss at 120±5 °C to 220±5 °C (the weight loss percentage is the proportion of the weight loss to the weight of the sample before the weight loss).

In another preferred embodiment, crystalline form 2 has a thermogravimetric analysis pattern with a 7.93%±0.5% weight loss at 120±1 °C (the weight loss percentage is the proportion of the weight loss to the weight of the sample before the weight loss) and a 7.24%±0.5% weight loss at 120±1 °C to 220±1 °C (the weight loss percentage is the proportion of the weight loss to the weight of the sample before the weight loss).

In another preferred embodiment, crystalline form 2 has a thermogravimetric analysis pattern substantially shown in FIG. 6.

In another preferred embodiment, the polymorph is crystalline form 3 of compound 1 having a characteristic X-ray powder diffraction peak at a 2• value selected from: 10.05±0.2°, 11.38±0.2°, 11.97±0.2°, 12.20±0.2°, 12.58±0.2°, 17.37±0.2°, 18.20±0.2°, 20.62±0.2°, 20.85±0.2°, and 24.95±0.2°.

In another preferred embodiment, crystalline form 3 has a characteristic X-ray powder diffraction peak at a 2• value selected from: 10.05±0.2°, 11.38±0.2°, 11.97±0.2°, 12.20±0.2°, 12.58±0.2°, 13.32±0.2°, 13.67±0.2°, 17.37±0.2°, 18.20±0.2°, 18.98±0.2°, 20.62±0.2°, 20.85±0.2°, and 24.95±0.2°.

In another preferred embodiment, crystalline form 3 has a characteristic X-ray powder diffraction peak at a 2• value selected from: 10.05±0.2°, 11.38±0.2°, 11.97±0.2°, 12.20±0.2°, 12.58±0.2°, 13.32±0.2°, 13.67±0.2°, 15.61±0.2°, 17.37±0.2°, 18.20±0.2°, 18.98±0.2°, 19.62±0.2°, 20.62±0.2°, 20.85±0.2°, and 24.95±0.2°.

In another preferred embodiment, crystalline form 3 has a characteristic X-ray powder diffraction peak at a 2• value selected from: 10.05±0.2°, 11.38±0.2°, 11.97±0.2°, 12.20±0.2°, 12.58±0.2°, 13.32±0.2°, 13.67±0.2°, 15.14±0.2°, 15.61±0.2°, 17.37±0.2°, 18.20±0.2°, 18.98±0.2°, 19.62±0.2°, 19.73±0.2°, 20.21±0.2°, 20.62±0.2°, 20.85±0.2°, 21.53±0.2°, 23.74±0.2°, 24.70±0.2°, 24.95±0.2°, 25.29±0.2°, 25.91±0.2°, 26.23±0.2°, 29.94±0.2°, and 30.57±0.2°.

In another preferred embodiment, crystalline form 3 has characteristic X-ray powder diffraction peaks at one or more (e.g., 2, 3, 4, 5, 6, 7, 8, 9 or more or all) 2• values selected from Table 4.

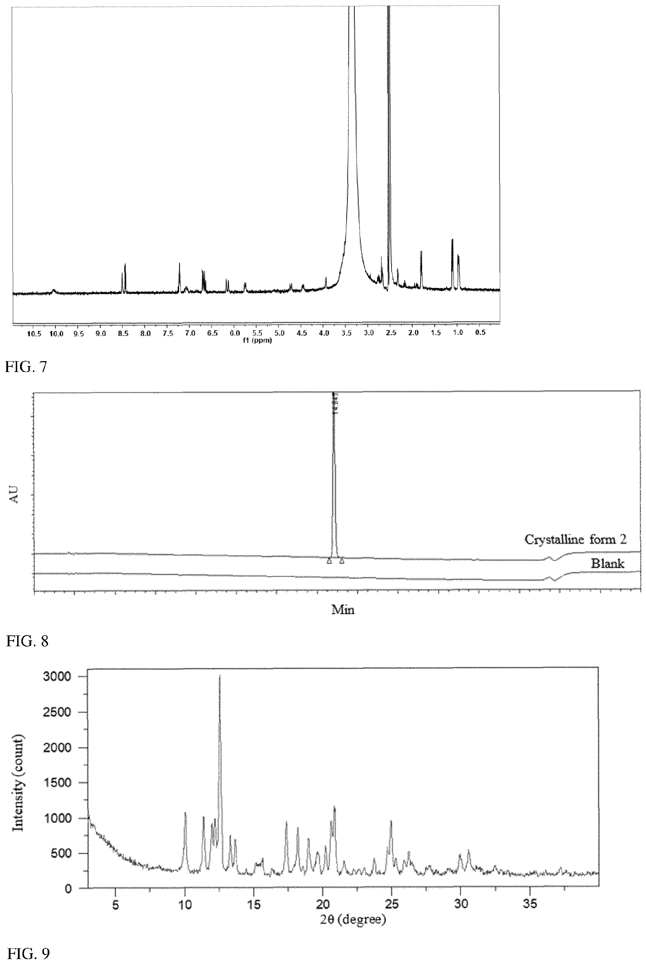

In another preferred embodiment, crystalline form 3 has an X-ray powder diffraction pattern substantially shown in FIG. 9.

In another preferred embodiment, crystalline form 3 has a differential scanning calorimetry pattern with a characteristic peak at 292.1±5 °C (endothermic peak).

In another preferred embodiment, crystalline form 3 has a differential scanning calorimetry pattern with a characteristic peak at 292.1±1 °C (endothermic peak).

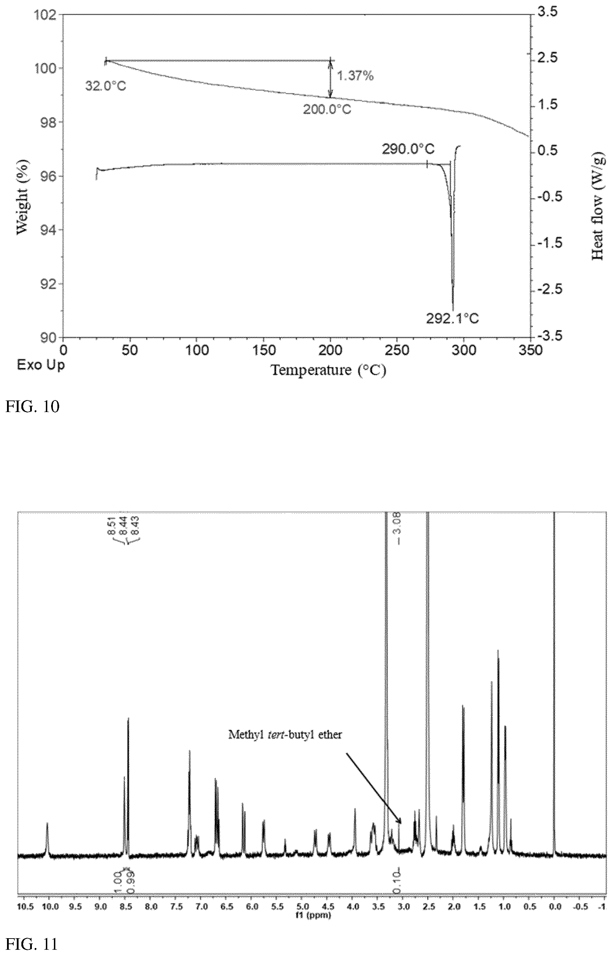

In another preferred embodiment, crystalline form 3 has a differential scanning calorimetry pattern substantially shown in FIG. 10.

In another preferred embodiment, crystalline form 3 has a thermogravimetric analysis pattern with a 1.37%±0.4% weight loss at 200±5 °C (the weight loss percentage is the proportion of the weight loss to the weight of the sample before the weight loss).

In another preferred embodiment, crystalline form 3 has a thermogravimetric analysis pattern with a 1.37%±0.2% weight loss at 200±1 °C (the weight loss percentage is the proportion of the weight loss to the weight of the sample before the weight loss).

In another preferred embodiment, crystalline form 3 has a thermogravimetric analysis pattern substantially shown in FIG. 10.

In another preferred embodiment, the polymorph is crystalline form 4 of compound 1 having a characteristic X-ray powder diffraction peak at a 2• value selected from: 6.00±0.2°, 6.96±0.2°, 12.11±0.2°, 13.04±0.2°, 17.09±0.2°, 17.40±0.2°, 19.66±0.2°, 21.63±0.2°, 23.24±0.2°, and 24.55±0.2°.

In another preferred embodiment, crystalline form 4 has a characteristic X-ray powder diffraction peak at a 2• value selected from: 6.00±0.2°, 6.96±0.2°, 12.11±0.2°, 13.04±0.2°, 13.96±0.2°, 17.09±0.2°, 17.40±0.2°, 18.80±0.2°, 19.66±0.2°, 21.63±0.2°, 23.12±0.2°, 23.24±0.2°, 24.55±0.2°, 25.56±0.2°, 26.01±0.2°, and 26.90±0.2°.

In another preferred embodiment, crystalline form 4 has a characteristic X-ray powder diffraction peak at a 2• value selected from: 6.00±0.2°, 6.96±0.2°, 12.11±0.2°, 13.04±0.2°, 13.96±0.2°, 14.27±0.2°, 14.71±0.2°, 16.28±0.2°, 17.09±0.2°, 17.40±0.2°, 18.8±0.2°, 19.66±0.2°, 19.90±0.2°, 20.81±0.2°, 21.07±0.2°, 21.63±0.2°, 22.59±0.2°, 23.12±0.2°, 23.24±0.2°, 24.33±0.2°, 24.55±0.2°, 25.56±0.2°, 26.01±0.2°, 26.9±0.2°, 27.95±0.2°, 28.49±0.2°, 29.05±0.2°, and 31.32±0.2°.

In another preferred embodiment, crystalline form 4 has characteristic X-ray powder diffraction peaks at one or more (e.g., 2, 3, 4, 5, 6, 7, 8, 9 or more or all) 2• values selected from Table 5.

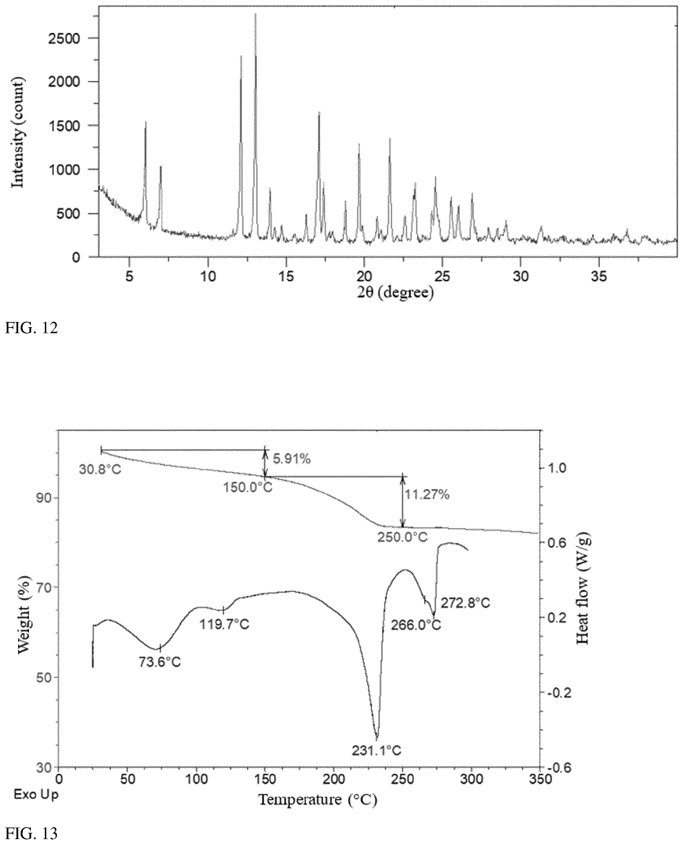

In another preferred embodiment, crystalline form 4 has an X-ray powder diffraction pattern substantially shown in FIG. 12.

In another preferred embodiment, crystalline form 4 has a differential scanning calorimetry pattern with characteristic peaks at 73.6±5 °C, 119.7±5 °C, 231.1±5 °C, 266.0±5 °C, and 272.8±5 °C (endothermic peaks).

In another preferred embodiment, crystalline form 4 has a differential scanning calorimetry pattern with characteristic peaks at 73.6±1 °C, 119.7±1 °C, 231.1±1 °C, 266.0±1 °C, and 272.8±1 °C (endothermic peaks).

In another preferred embodiment, crystalline form 4 has a differential scanning calorimetry pattern substantially shown in FIG. 13.

In another preferred embodiment, crystalline form 4 has a thermogravimetric analysis pattern with a 5.91%±1% weight loss at 150±5 °C (the weight loss percentage is the proportion of the weight loss to the weight of the sample before the weight loss) and an 11.27%±1% weight loss at 150±5 °C to 250±5 °C (the weight loss percentage is the proportion of the weight loss to the weight of the sample before the weight loss).

In another preferred embodiment, crystalline form 4 has a thermogravimetric analysis pattern with a 5.91%±0.5% weight loss at 150±1 °C (the weight loss percentage is the proportion of the weight loss to the weight of the sample before the weight loss) and an 11.27%±0.5% weight loss at 150±1 °C to 250±1 °C (the weight loss percentage is the proportion of the weight loss to the weight of the sample before the weight loss).

In another preferred embodiment, crystalline form 4 has a thermogravimetric analysis pattern substantially shown in FIG. 13.

In another preferred embodiment, the polymorph is crystalline form 5 of compound 1 having a characteristic X-ray powder diffraction peak at a 2• value selected from: 11.98±0.2°, 12.33±0.2°, 13.62±0.2°, 14.86±0.2°, 16.00±0.2°, 17.88±0.2°, 19.05±0.2°, 21.91±0.2°, 23.42±0.2°, 25.32±0.2°, and 26.04±0.2°.

In another preferred embodiment, crystalline form 5 has a characteristic X-ray powder diffraction peak at a 2• value selected from: 11.98±0.2°, 12.33±0.2°, 13.62±0.2°, 14.86±0.2°, 16.00±0.2°, 17.88±0.2°, 19.05±0.2°, 19.71±0.2°, 20.67±0.2°, 21.91±0.2°, 23.42±0.2°, 24.33±0.2°, 25.32±0.2°, and 26.04±0.2°.

In another preferred embodiment, crystalline form 5 has a characteristic X-ray powder diffraction peak at a 2• value selected from: 6.9±0.2°, 11.98±0.2°, 12.33±0.2°, 13.62±0.2°, 14.86±0.2°, 16.00±0.2°, 17.10±0.2°, 17.88±0.2°, 19.05±0.2°, 19.71±0.2°, 20.67±0.2°, 21.91±0.2°, 23.42±0.2°, 24.33±0.2°, 24.80±0.2°, 25.32±0.2°, 26.04±0.2°, 26.76±0.2°, 28.18±0.2°, and 28.99±0.2°.

In another preferred embodiment, crystalline form 5 has characteristic X-ray powder diffraction peaks at one or more (e.g., 2, 3, 4, 5, 6, 7, 8, 9 or more or all) 2• values selected from Table 6.

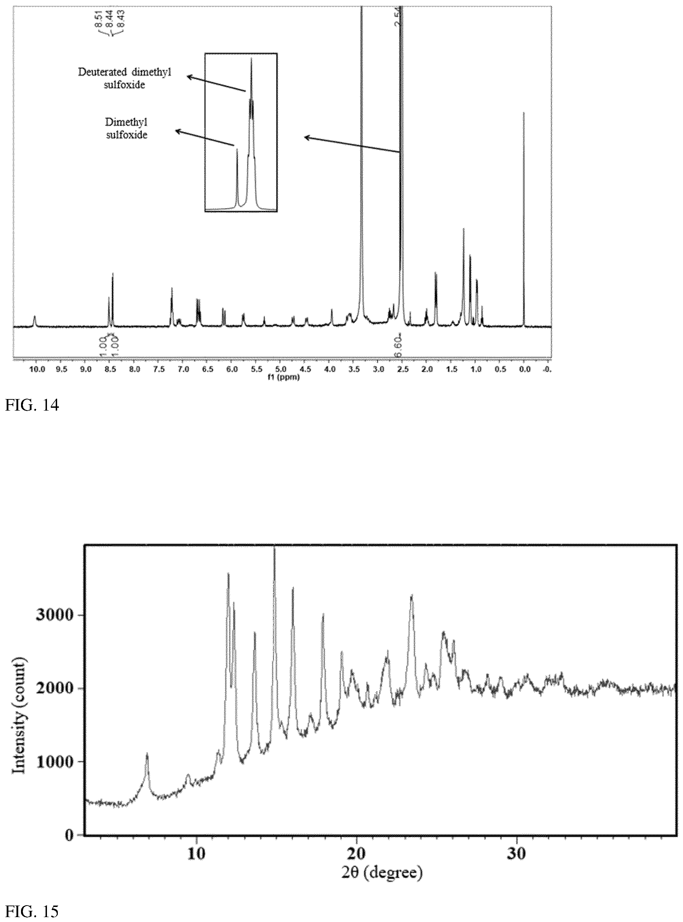

In another preferred embodiment, crystalline form 5 has an X-ray powder diffraction pattern substantially shown in FIG. 15.

In another preferred embodiment, crystalline form 5 has a differential scanning calorimetry pattern with a characteristic peak at 275.8±5 °C (endothermic peak).

In another preferred embodiment, crystalline form 5 has a differential scanning calorimetry pattern with a characteristic peak at 275.8±1 °C (endothermic peak).

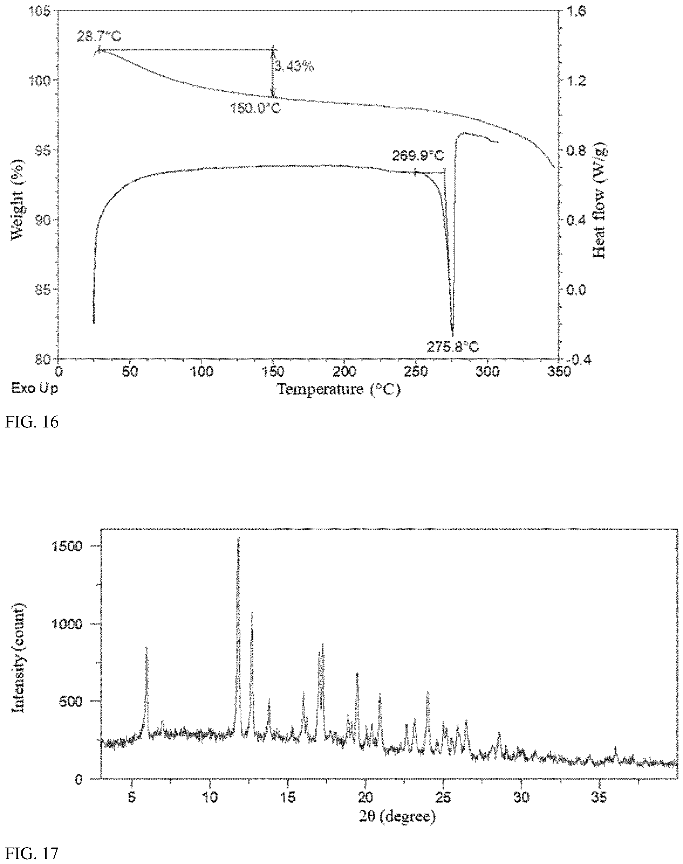

In another preferred embodiment, crystalline form 5 has a differential scanning calorimetry pattern substantially shown in FIG. 16.

In another preferred embodiment, crystalline form 5 has a thermogravimetric analysis pattern with a 3.43%±0.5% weight loss at 150±5 °C (the weight loss percentage is the proportion of the weight loss to the weight of the sample before the weight loss).

In another preferred embodiment, crystalline form 5 has a thermogravimetric analysis pattern with a 3.43%±0.2% weight loss at 150±1 °C (the weight loss percentage is the proportion of the weight loss to the weight of the sample before the weight loss).

In another preferred embodiment, crystalline form 5 has a thermogravimetric analysis pattern substantially shown in FIG. 16.

In another preferred embodiment, the polymorph is crystalline form 6 of compound 1 having a characteristic X-ray powder diffraction peak at a 2• value selected from: 5.94±0.2°, 11.80±0.2°, 12.70±0.2°, 17.02±0.2°, 17.25±0.2°, 19.45±0.2°, and 23.99±0.2°.

In another preferred embodiment, crystalline form 6 has a characteristic X-ray powder diffraction peak at a 2• value selected from: 5.94±0.2°, 11.80±0.2°, 12.70±0.2°, 13.79±0.2°, 16.00±0.2°, 17.02±0.2°, 17.25±0.2°, 19.45±0.2°, 20.92±0.2°, 23.16±0.2°, 23.99±0.2°, 25.89±0.2°, and 26.46±0.2°.

In another preferred embodiment, crystalline form 6 has a characteristic X-ray powder diffraction peak at a 2• value selected from: 5.94±0.2°, 6.97±0.2°, 11.80±0.2°, 12.70±0.2°, 13.79±0.2°, 16.00±0.2°, 16.23±0.2°, 17.02±0.2°, 17.25±0.2°, 18.86±0.2°, 19.09±0.2°, 19.45±0.2°, 20.07±0.2°, 20.40±0.2°, 20.92±0.2°, 22.64±0.2°, 23.16±0.2°, 23.99±0.2°, 25.01±0.2°, 25.20±0.2°, 25.55±0.2°, 25.89±0.2°, 26.46±0.2°, and 28.58±0.2°.

In another preferred embodiment, crystalline form 6 has characteristic X-ray powder diffraction peaks at one or more (e.g., 2, 3, 4, 5, 6, 7, 8, 9 or more or all) 2• values selected from Table 7.

In another preferred embodiment, crystalline form 6 has an X-ray powder diffraction pattern substantially shown in FIG. 17.

In another preferred embodiment, crystalline form 6 has a differential scanning calorimetry pattern with characteristic peaks at 104.0±5 °C and 279.1±5 °C (endothermic peaks) and a characteristic peak at 213.1±5 °C (exothermic peak).

In another preferred embodiment, crystalline form 6 has a differential scanning calorimetry pattern with characteristic peaks at 104.0±1 °C and 279.1±1 °C (endothermic peaks) and a characteristic peak at 213.1±1 °C (exothermic peak).

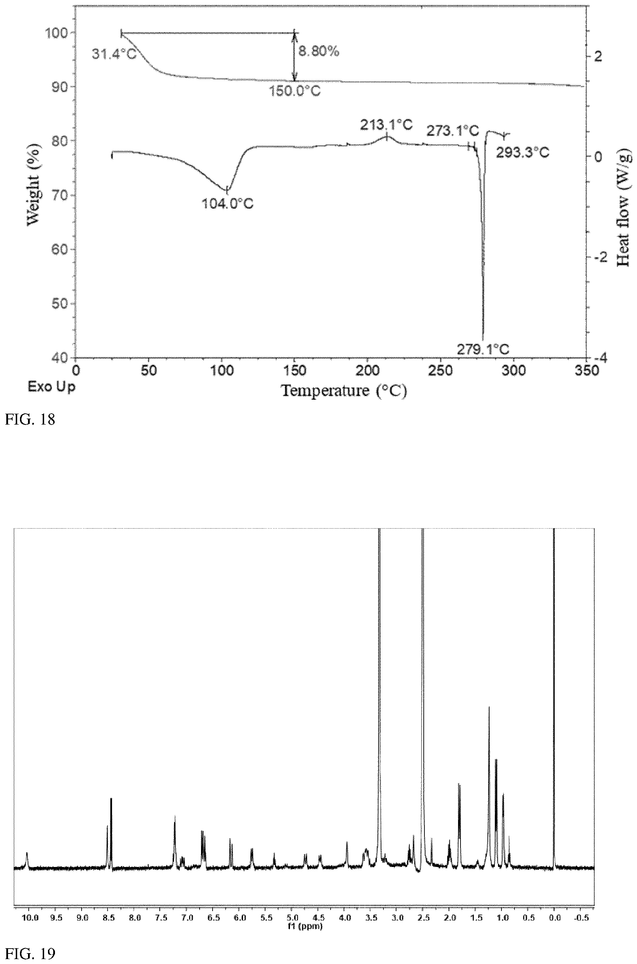

In another preferred embodiment, crystalline form 6 has a differential scanning calorimetry pattern substantially shown in FIG. 18.

In another preferred embodiment, crystalline form 6 has a thermogravimetric analysis pattern with an 8.80%±2% weight loss at 150±5 °C. (the weight loss percentage is the proportion of the weight loss to the weight of the sample before the weight loss).

In another preferred embodiment, crystalline form 6 has a thermogravimetric analysis pattern with an 8.80%±0.5% weight loss at 150±1 °C. (the weight loss percentage is the proportion of the weight loss to the weight of the sample before the weight loss).

In another preferred embodiment, crystalline form 6 has a thermogravimetric analysis pattern substantially shown in FIG. 18.

In another preferred embodiment, the polymorph is crystalline form 7 of compound 1 having a characteristic X-ray powder diffraction peak at a 2• value selected from: 6.33±0.2°, 11.60±0.2°, 13.01±0.2°, 13.51±0.2°, 16.54±0.2°, 17.38±0.2°, 18.12±0.2°, 20.20±0.2°, and 23.01±0.2°.

In another preferred embodiment, crystalline form 7 has a characteristic X-ray powder diffraction peak at a 2• value selected from: 6.33±0.2°, 11.38±0.2°, 11.60±0.2°, 12.77±0.2°, 13.01±0.2°, 13.51±0.2°, 16.54±0.2°, 17.38±0.2°, 18.12±0.2°, 19.00±0.2°, 20.20±0.2°, 23.01±0.2°, and 24.04±0.2°.

In another preferred embodiment, crystalline form 7 has a characteristic X-ray powder diffraction peak at a 2• value selected from: 6.33±0.2°, 11.38±0.2°, 11.60±0.2°, 12.77±0.2°, 13.01±0.2°, 13.51±0.2°, 15.92±0.2°, 16.54±0.2°, 17.38±0.2°, 18.12±0.2°, 19.00±0.2°, 20.20±0.2°, 22.12±0.2°, 23.01±0.2°, 24.04±0.2°, 24.60±0.2°, 25.01±0.2°, 25.23±0.2°, and 27.32±0.2°.

In another preferred embodiment, crystalline form 7 has characteristic X-ray powder diffraction peaks at one or more (e.g., 2, 3, 4, 5, 6, 7, 8, 9 or more or all) 2• values selected from Table 8.

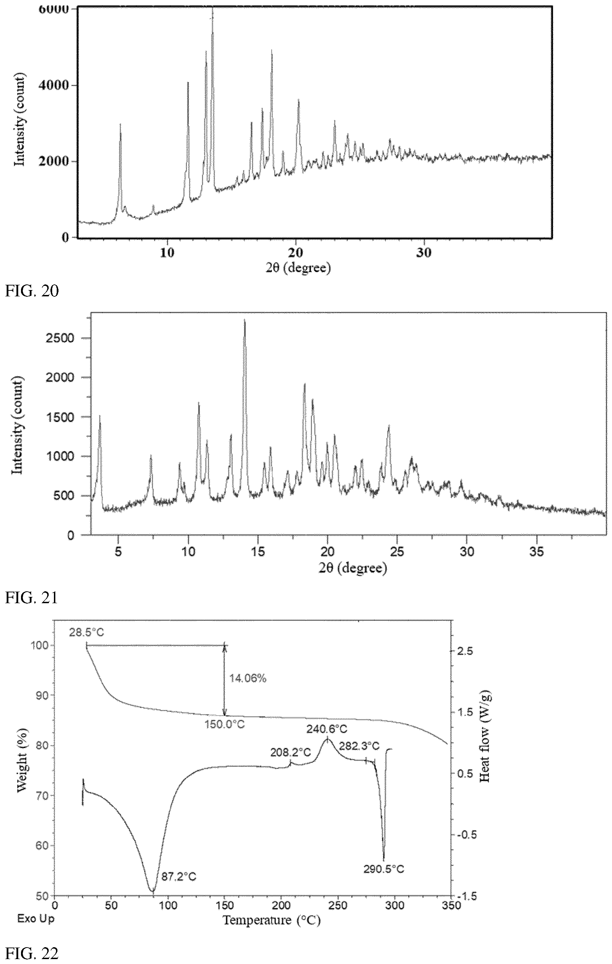

In another preferred embodiment, crystalline form 7 has an X-ray powder diffraction pattern substantially shown in FIG. 20.

In another preferred embodiment, the polymorph is crystalline form 8 of compound 1 having a characteristic X-ray powder diffraction peak at a 2• value selected from: 3.64±0.2°, 10.74±0.2°, 11.36±0.2°, 13.04±0.2°, 14.06±0.2°, 15.89±0.2°, 18.32±0.2°, 18.93±0.2°, 19.97±0.2°, 20.50±0.2°, and 24.40±0.2°.

In another preferred embodiment, crystalline form 8 has a characteristic X-ray powder diffraction peak at a 2• value selected from: 3.64±0.2°, 7.32±0.2°, 10.74±0.2°, 11.36±0.2°, 13.04±0.2°, 14.06±0.2°, 15.46±0.2°, 15.89±0.2°, 18.32±0.2°, 18.93±0.2°, 19.60±0.2°, 19.97±0.2°, 20.50±0.2°, 21.97±0.2°, 22.43±0.2°, 23.84±0.2°, 24.40±0.2°, 26.04±0.2°, and 26.36±0.2°.

In another preferred embodiment, crystalline form 8 has a characteristic X-ray powder diffraction peak at a 2• value selected from: 3.64±0.2°, 7.32±0.2°, 9.39±0.2°, 10.74±0.2°, 11.36±0.2°, 13.04±0.2°, 14.06±0.2°, 15.46±0.2°, 15.89±0.2°, 17.14±0.2°, 17.80±0.2°, 18.32±0.2°, 18.93±0.2°, 19.60±0.2°, 19.97±0.2°, 20.50±0.2°, 21.12±0.2°, 21.97±0.2°, 22.43±0.2°, 22.93±0.2°, 23.84±0.2°, 24.40±0.2°, 24.83±0.2°, 25.58±0.2°, 26.04±0.2°, 26.36±0.2°, 27.20±0.2°, 27.52±0.2°, 28.7±0.2°, and 29.59±0.2°.

In another preferred embodiment, crystalline form 8 has characteristic X-ray powder diffraction peaks at one or more (e.g., 2, 3, 4, 5, 6, 7, 8, 9 or more or all) 2• values selected from Table 9.

In another preferred embodiment, crystalline form 8 has an X-ray powder diffraction pattern substantially shown in FIG. 21.

In another preferred embodiment, crystalline form 8 has a differential scanning calorimetry pattern with characteristic peaks at 87.2±5 °C and 290.5±5 °C (endothermic peaks) and characteristic peaks at 208.2±5 °C and 240.6±5 °C (exothermic peaks).

In another preferred embodiment, crystalline form 8 has a differential scanning calorimetry pattern with characteristic peaks at 87.2±1 °C and 290.5±1 °C (endothermic peaks) and characteristic peaks at 208.2±1 °C and 240.6±1 °C (exothermic peaks).

In another preferred embodiment, crystalline form 8 has a differential scanning calorimetry pattern substantially shown in FIG. 22.

In another preferred embodiment, crystalline form 8 has a thermogravimetric analysis pattern with a 14.06%±2% weight loss at 150±5 °C. (the weight loss percentage is the proportion of the weight loss to the weight of the sample before the weight loss).

In another preferred embodiment, crystalline form 8 has a thermogravimetric analysis pattern with a 14.06%±0.5% weight loss at 150±1 °C. (the weight loss percentage is the proportion of the weight loss to the weight of the sample before the weight loss).

In another preferred embodiment, crystalline form 8 has a thermogravimetric analysis pattern substantially shown in FIG. 22.

In another preferred embodiment, the polymorph is crystalline form 9 of compound 1 having a characteristic X-ray powder diffraction peak at a 2• value selected from: 3.36±0.2°, 7.98±0.2°, 9.90±0.2°, 14.66±0.2°, 15.48±0.2°, 16.87±0.2°, 22.25±0.2°, and 27.25±0.2°.

In another preferred embodiment, crystalline form 9 has a characteristic X-ray powder diffraction peak at a 2• value selected from: 3.36±0.2°, 7.98±0.2°, 9.90±0.2°, 14.66±0.2°, 15.48±0.2°, 16.87±0.2°, 20.24±0.2°, 22.25±0.2°, 26.33±0.2°, and 27.25±0.2°.

In another preferred embodiment, crystalline form 9 has a characteristic X-ray powder diffraction peak at a 2• value selected from: 3.36±0.2°, 7.98±0.2°, 8.86±0.2°, 9.90±0.2°, 13.86±0.2°, 14.66±0.2°, 15.48±0.2°, 16.87±0.2°, 20.24±0.2°, 22.25±0.2°, 23.96±0.2°, 26.33±0.2°, 27.25±0.2°, 28.55±0.2°, and 30.68±0.2°.

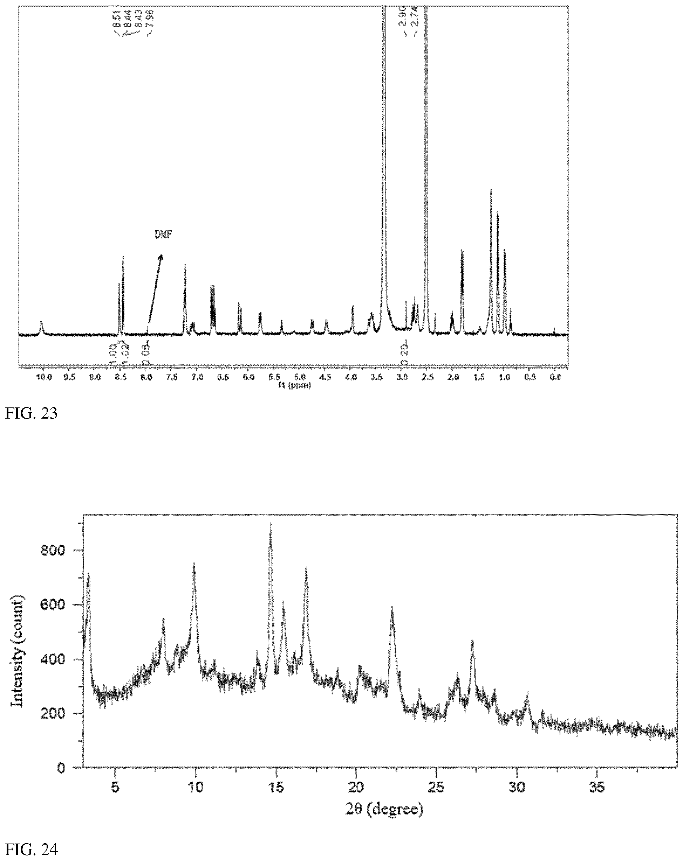

In another preferred embodiment, crystalline form 9 has characteristic X-ray powder diffraction peaks at one or more (e.g., 2, 3, 4, 5, 6, 7, 8, 9 or more or all) 2• values selected from Table 10. In another preferred embodiment, crystalline form 9 has an X-ray powder diffraction pattern substantially shown in FIG. 24.

In another preferred embodiment, crystalline form 9 has a differential scanning calorimetry pattern with characteristic peaks at 84.3±5 °C, 119.8±5 °C, 194.7±5 °C, 214.4±5 °C, and 289.7±5 °C (endothermic peaks).

In another preferred embodiment, crystalline form 9 has a differential scanning calorimetry pattern with characteristic peaks at 84.3±1 °C, 119.8±1 °C, 194.7±1 °C, 214.4±1 °C, and 289.7±1 °C.

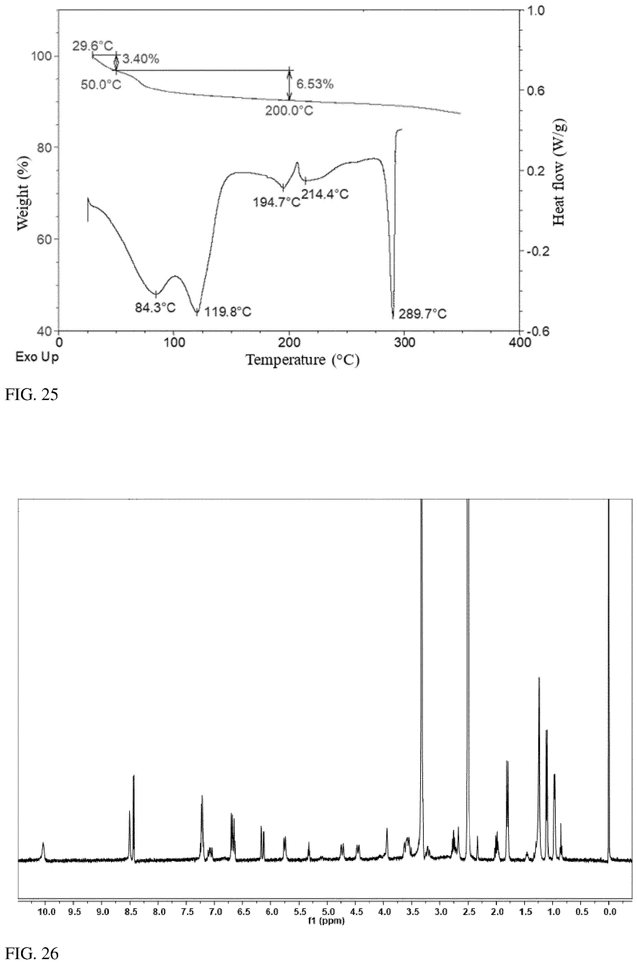

In another preferred embodiment, crystalline form 9 has a differential scanning calorimetry pattern substantially shown in FIG. 25.

In another preferred embodiment, crystalline form 9 has a thermogravimetric analysis pattern with a 3.40%±0.5% weight loss at 50±5 °C (the weight loss percentage is the proportion of the weight loss to the weight of the sample before the weight loss) and a 6.53%±1% weight loss at 50±5 °C to 200±5 °C (the weight loss percentage is the proportion of the weight loss to the weight of the sample before the weight loss).

In another preferred embodiment, crystalline form 9 has a thermogravimetric analysis pattern with a 3.40%±0.2% weight loss at 50±1 °C (the weight loss percentage is the proportion of the weight loss to the weight of the sample before the weight loss) and a 6.53%±0.5% weight loss at 50±1 °C to 200±1 °C (the weight loss percentage is the proportion of the weight loss to the weight of the sample before the weight loss).

In another preferred embodiment, crystalline form 9 has a thermogravimetric analysis pattern substantially shown in FIG. 25.

In another preferred embodiment, the polymorph is crystalline form 10 of compound 1 having a characteristic X-ray powder diffraction peak at a 2• value selected from: 10.03±0.2°, 13.8±0.2°, 14.49±0.2°, 15.42±0.2°, 17.01±0.2°, and 17.45±0.2°.

In another preferred embodiment, crystalline form 10 has a characteristic X-ray powder diffraction peak at a 2• value selected from: 10.03±0.2°, 13.8±0.2°, 14.49±0.2°, 15.42±0.2°, 17.01±0.2°, 17.45±0.2°, 19.21±0.2°, and 22.14±0.2°.

In another preferred embodiment, crystalline form 10 has a characteristic X-ray powder diffraction peak at a 2• value selected from: 7.97±0.2°, 10.03±0.2°, 12.69±0.2°, 13.8±0.2°, 14.49±0.2°, 15.42±0.2°, 17.01±0.2°, 17.45±0.2°, 19.21±0.2°, 20.56±0.2°, 22.14±0.2°, and 24.59±0.2°.

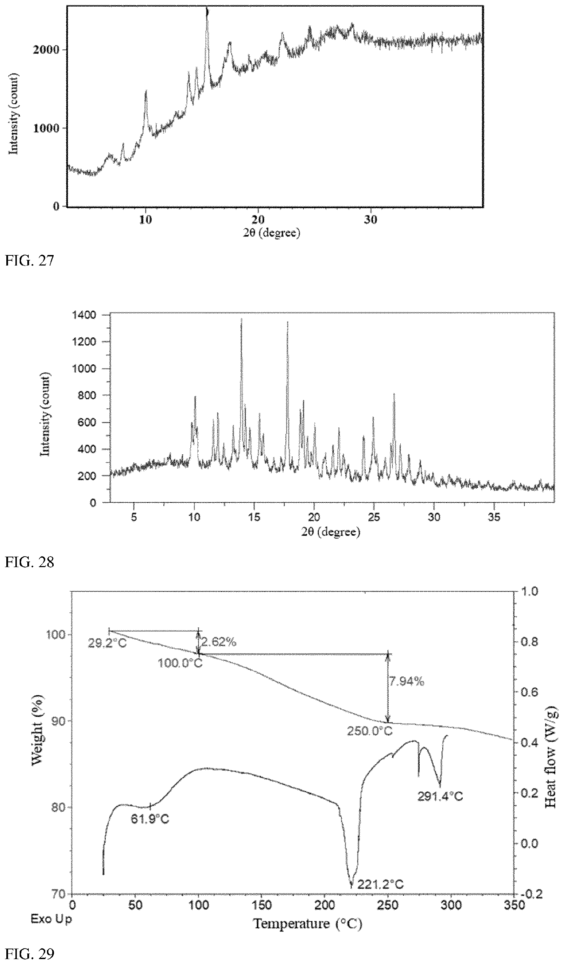

In another preferred embodiment, crystalline form 10 has characteristic X-ray powder diffraction peaks at one or more (e.g., 2, 3, 4, 5, 6, 7, 8, 9 or more or all) 2• values selected from Table 11. In another preferred embodiment, crystalline form 10 has an X-ray powder diffraction pattern substantially shown in FIG. 27.

In another preferred embodiment, the polymorph is crystalline form 11 of compound 1 having a characteristic X-ray powder diffraction peak at a 2• value selected from: 10.07±0.2°, 11.60±0.2°, 11.97±0.2°, 13.94±0.2°, 14.26±0.2°, 15.43±0.2°, 17.78±0.2°, 18.85±0.2°, 19.09±0.2°, 20.06±0.2°, 24.93±0.2°, and 26.67±0.2°.

In another preferred embodiment, crystalline form 11 has a characteristic X-ray powder diffraction peak at a 2• value selected from: 9.83±0.2°, 10.07±0.2°, 10.24±0.2°, 11.60±0.2°, 11.97±0.2°, 13.26±0.2°, 13.94±0.2°, 14.26±0.2°, 14.65±0.2°, 15.43±0.2°, 15.75±0.2°, 17.78±0.2°, 18.85±0.2°, 19.09±0.2°, 20.06±0.2°, 22.05±0.2°, 24.12±0.2°, 24.93±0.2°, and 26.67±0.2°.

In another preferred embodiment, crystalline form 11 has a characteristic X-ray powder diffraction peak at a 2• value selected from: 9.83±0.2°, 10.07±0.2°, 10.24±0.2°, 11.60±0.2°, 11.97±0.2°, 12.46±0.2°, 13.26±0.2°, 13.94±0.2°, 14.26±0.2°, 14.65±0.2°, 15.43±0.2°, 15.75±0.2°, 17.78±0.2°, 18.85±0.2°, 19.09±0.2°, 19.43±0.2°, 20.06±0.2°, 22.05±0.2°, 24.12±0.2°, 24.93±0.2°, 25.21±0.2°, 26.41±0.2°, 26.67±0.2°, 27.18±0.2°, and 27.93±0.2°.

In another preferred embodiment, crystalline form 11 has characteristic X-ray powder diffraction peaks at one or more (e.g., 2, 3, 4, 5, 6, 7, 8, 9 or more or all) 2• values selected from Table 12. In another preferred embodiment, crystalline form 11 has an X-ray powder diffraction pattern substantially shown in FIG. 28.

In another preferred embodiment, crystalline form 11 has a differential scanning calorimetry pattern with characteristic peaks at 61.9±5 °C, 221.2±5 °C, and 291.4±5 °C (endothermic peaks). In another preferred embodiment, crystalline form 11 has a differential scanning calorimetry pattern with characteristic peaks at 61.9±1 °C, 221.2±1 °C, and 291.4±1 °C (endothermic peaks). In another preferred embodiment, crystalline form 11 has a differential scanning calorimetry pattern substantially shown in FIG. 29.

In another preferred embodiment, crystalline form 11 has a thermogravimetric analysis pattern with a 2.62%±0.5% weight loss at 100±5 °C (the weight loss percentage is the proportion of the weight loss to the weight of the sample before the weight loss) and a 7.94%±1% weight loss at 100±5 °C to 250±5 °C (the weight loss percentage is the proportion of the weight loss to the weight of the sample before the weight loss).

In another preferred embodiment, crystalline form 11 has a thermogravimetric analysis pattern with a 2.62%±0.2% weight loss at 100±1 °C (the weight loss percentage is the proportion of the weight loss to the weight of the sample before the weight loss) and a 7.94%±0.5% weight loss at 100±1 °C to 250±1 °C (the weight loss percentage is the proportion of the weight loss to the weight of the sample before the weight loss).

In another preferred embodiment, crystalline form 11 has a thermogravimetric analysis pattern substantially shown in FIG. 29.

In another preferred embodiment, the polymorph is crystalline form 12 of compound 1 having a characteristic X-ray powder diffraction peak at a 2• value selected from: 5.77±0.2°, 6.21±0.2°, 11.75±0.2°, 12.07±0.2°, 12.43±0.2°, 12.78±0.2°, 14.29±0.2°, 14.93±0.2°, 16.89±0.2°, 17.78±0.2°, 18.01±0.2°, 18.92±0.2°, 19.34±0.2°, 20.19±0.2°, 23.01±0.2°, 23.67±0.2°, and 25.86±0.2°.

In another preferred embodiment, crystalline form 12 has a characteristic X-ray powder diffraction peak at a 2• value selected from: 5.77±0.2°, 6.21±0.2°, 11.75±0.2°, 12.07±0.2°, 12.43±0.2°, 12.78±0.2°, 14.29±0.2°, 14.93±0.2°, 16.89±0.2°, 17.16±0.2°, 17.78±0.2°, 18.01±0.2°, 18.92±0.2°, 19.34±0.2°, 20.19±0.2°, 23.01±0.2°, 23.67±0.2°, 25.18±0.2°, 25.86±0.2°, 26.73±0.2°, and 28.87±0.2°.

In another preferred embodiment, crystalline form 12 has a characteristic X-ray powder diffraction peak at a 2• value selected from: 5.77±0.2°, 6.21±0.2°, 7.71±0.2°, 11.75±0.2°, 12.07±0.2°, 12.43±0.2°, 12.78±0.2°, 14.29±0.2°, 14.55±0.2°, 14.93±0.2°, 16.20±0.2°, 16.89±0.2°, 17.16±0.2°, 17.78±0.2°, 18.01±0.2°, 18.92±0.2°, 19.34±0.2°, 20.19±0.2°, 21.03±0.2°, 21.44±0.2°, 22.18±0.2°, 23.01±0.2°, 23.67±0.2°, 24.86±0.2°, 25.18±0.2°, 25.86±0.2°, 26.73±0.2°, 28.87±0.2°, and 30.33±0.2°.

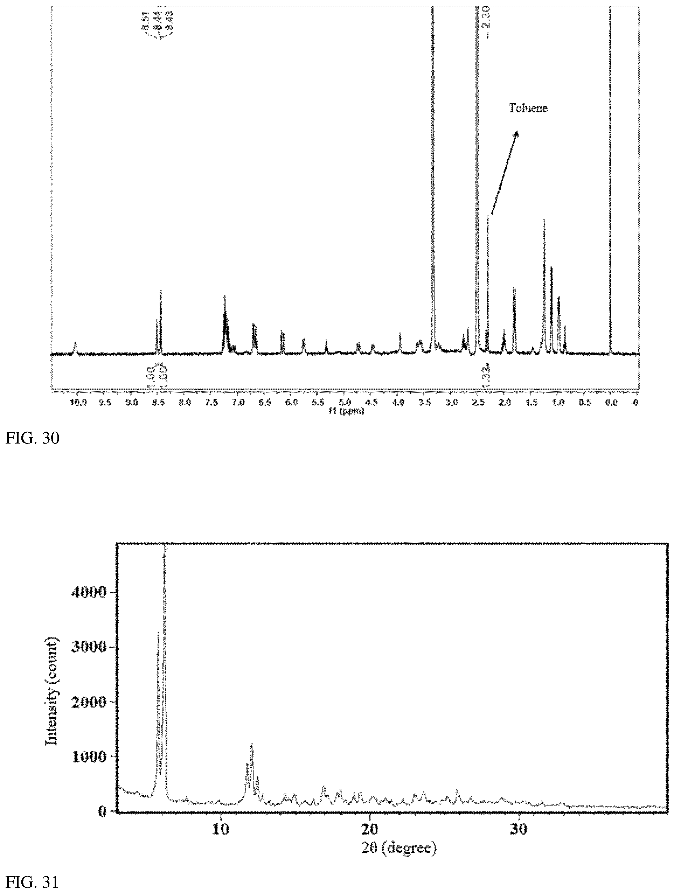

In another preferred embodiment, crystalline form 12 has characteristic X-ray powder diffraction peaks at one or more (e.g., 2, 3, 4, 5, 6, 7, 8, 9 or more or all) 2• values selected from Table 13. In another preferred embodiment, crystalline form 12 has an X-ray powder diffraction pattern substantially shown in FIG. 31.

In another preferred embodiment, crystalline form 12 has a differential scanning calorimetry pattern with characteristic peaks at 125.1±5 °C and 291.8±5 °C (endothermic peaks).

In another preferred embodiment, crystalline form 12 has a differential scanning calorimetry pattern with characteristic peaks at 125.1±1 °C and 291.8±1 °C (endothermic peaks).

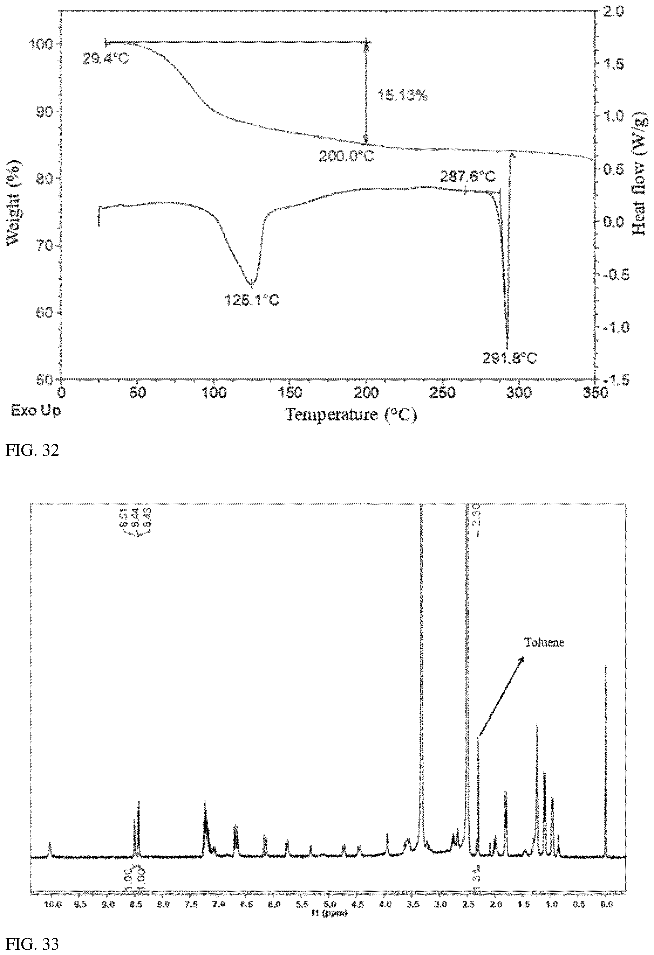

In another preferred embodiment, crystalline form 12 has a differential scanning calorimetry pattern substantially shown in FIG. 32.

In another preferred embodiment, crystalline form 12 has a thermogravimetric analysis pattern with a 15.13%±2% weight loss at 200±5 °C (the weight loss percentage is the proportion of the weight loss to the weight of the sample before the weight loss).

In another preferred embodiment, crystalline form 12 has a thermogravimetric analysis pattern with a 15.13%±0.5% weight loss at 200±1 °C (the weight loss percentage is the proportion of the weight loss to the weight of the sample before the weight loss).

In another preferred embodiment, crystalline form 12 has a thermogravimetric analysis pattern substantially shown in FIG. 32.

In another preferred embodiment, the polymorph is crystalline form 13 of compound 1 having a characteristic X-ray powder diffraction peak at a 2• value selected from: 6.16±0.2°, 11.94±0.2°, 12.45±0.2°, 16.97±0.2°, 18.10±0.2°, 19.43±0.2°, 19.89±0.2°, and 23.52±0.2°.

In another preferred embodiment, crystalline form 13 has a characteristic X-ray powder diffraction peak at a 2• value selected from: 6.16±0.2°, 11.94±0.2°, 12.45±0.2°, 14.26±0.2°, 15.58±0.2°, 16.97±0.2°, 18.10±0.2°, 19.43±0.2°, 19.89±0.2°, 20.62±0.2°, 23.52±0.2°, 24.24±0.2°, 25.15±0.2°, 26.79±0.2°, and 27.83±0.2°.

In another preferred embodiment, crystalline form 13 has a characteristic X-ray powder diffraction peak at a 2• value selected from: 6.16±0.2°, 11.94±0.2°, 12.45±0.2°, 13.08±0.2°, 14.26±0.2°, 14.85±0.2°, 15.58±0.2°, 16.97±0.2°, 18.10±0.2°, 19.43±0.2°, 19.89±0.2°, 20.62±0.2°, 21.24±0.2°, 22.24±0.2°, 23.52±0.2°, 24.24±0.2°, 25.15±0.2°, 26.79±0.2°, 27.83±0.2°, and 30.24±0.2°.

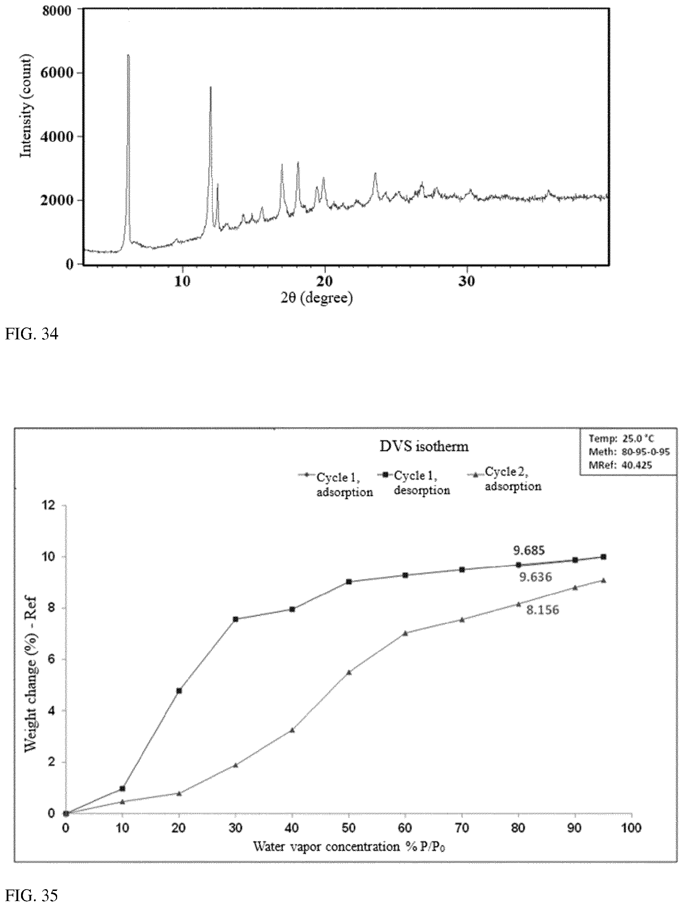

In another preferred embodiment, crystalline form 13 has characteristic X-ray powder diffraction peaks at one or more (e.g., 2, 3, 4, 5, 6, 7, 8, 9 or more or all) 2• values selected from Table 14. In another preferred embodiment, crystalline form 13 has an X-ray powder diffraction pattern substantially shown in FIG. 34.

In another preferred embodiment, the advantageous salt form of the pharmaceutically acceptable salt of the compound has improved properties in aspects such as stability and/or efficacy.

In another preferred embodiment, the advantageous crystalline form of the polymorph has improved properties in aspects such as stability, flowability, pressure resistance, and/or hygroscopicity.

In another preferred embodiment, the X-ray used in the X-ray powder diffraction (XRPD) analysis in the present invention is Cu, K•. For example, K•1(Å): 1.540598; K•2(Å): 1.544426; K•2/K•1 intensity ratio: 0.50.

In another preferred embodiment, the ramping rate for thermogravimetric analysis (TGA) in the present invention is 10 °C/min.

In another preferred embodiment, the ramping rate for differential scanning calorimetry (DSC) analysis in the present invention is 10 °C/min.

In a third aspect, the present invention provides a pharmaceutical composition, comprising: (a) the pharmaceutically acceptable salt of the compound according to the first aspect or the polymorph according to the second aspect; and (b) a pharmaceutically acceptable carrier.

In another aspect, the present invention provides use of the pharmaceutically acceptable salt of the compound according to the first aspect, the polymorph according to the second aspect, or the pharmaceutical composition according to the third aspect in preparing a medicament for preventing and/or treating a disease associated with KRAS G12C mutation.

In another preferred embodiment, the disease associated with KRAS G12C mutation is a cancer. In another preferred embodiment, the cancer is a solid tumor, for example, one or more cancers selected from: lung cancer (e.g., non-small cell lung cancer), pancreatic cancer, and colorectal cancer.

In another aspect, the present invention provides a method for preparing crystalline form 1 of compound 1 hydrochloride, comprising: stirring compound 1 with hydrochloric acid in a first solvent at 10-30 °C to give crystalline form 1 of compound 1 hydrochloride.

In another preferred embodiment, the first solvent is tetrahydrofuran.

In another aspect, the present invention provides a method for preparing crystalline form 2 of compound 1 phosphate, comprising: stirring compound 1 with phosphoric acid in a second solvent at 10-30 °C to give crystalline form 2 of compound 1 phosphate.

In another preferred embodiment, the second solvent is selected from: acetone, acetonitrile, a combination of methanol and water, or tetrahydrofuran.

In another aspect, the present invention provides a method for preparing crystalline form 3 of compound 1, wherein
the method comprises: suspending and stirring compound 1 or crystalline form 6 of compound 1 in a third solvent at 50±10 °C to give crystalline form 3 of compound 1; or
the method comprises: dissolving compound 1 in a first good solvent and adding a first anti-solvent while stirring to give crystalline form 3 of compound 1; or
the method comprises: heating crystalline form 8, crystalline form 10, crystalline form 11, or crystalline form 13 of compound 1 to 200-250 °C, and cooling to 10-30 °C to give crystalline form 3 of compound 1.

In another preferred embodiment, the third solvent is selected from: methyl tert-butyl ether, *n-*heptane, a combination of dichloromethane and n-heptane, or a combination of isopropyl acetate and n-hexane.

In another preferred embodiment, the first good solvent is methyl isobutyl ketone or chloroform; the first anti-solvent is n-heptane or methyl tert-butyl ether.

In another preferred embodiment, in the methods for preparing crystalline form 3, the heating procedures are each independently optionally conducted in a nitrogen atmosphere.

In another preferred embodiment, the methods for preparing crystalline form 3 each independently optionally further comprise, after cooling to 10-30 °C, drying the obtained solid at 10-30 °C.

In another aspect, the present invention provides a method for preparing crystalline form 4 of compound 1, wherein
the method comprises: subjecting compound 1 to gas-solid diffusion in a fourth solvent atmosphere at 10-30 °C to give crystalline form 4 of compound 1; or
the method comprises: dissolving compound 1 in a second good solvent and adding a second anti-solvent while stirring to give crystalline form 4 of compound 1.

In another preferred embodiment, the fourth solvent is dimethyl sulfoxide.

In another preferred embodiment, the second good solvent is dimethyl sulfoxide; the second anti-solvent is methyl *tert*-butyl ether.

In another aspect, the present invention provides a method for preparing crystalline form 5 of compound 1, comprising: in a nitrogen atmosphere, heating crystalline form 4 or crystalline form 7 of compound 1 to 200-300 °C and cooling to 10-30 °C to give crystalline form 5 of compound 1.

In another preferred embodiment, the heating temperature is 200-250 °C or 220-250 °C.

In another aspect, the present invention provides a method for preparing crystalline form 6 of compound 1, wherein
the method comprises: stirring compound 1 in a fifth solvent in a temperature cycle to give crystalline form 6 of compound 1, the temperature cycle comprising stirring at 50 °C (e.g., for 1-3 h), cooling to 5 °C and stirring (e.g., for 0.1-1 h), and heating to 50 °C and stirring (e.g., for 0.1-1 h); or
the method comprises: suspending and stirring compound 1 in a sixth solvent at 10-50 °C to give crystalline form 6 of compound 1; or
the method comprises: subjecting compound 1 to gas-solid diffusion in a seventh solvent atmosphere at 10-30 °C to give crystalline form 6 of compound 1; or
the method comprises: dissolving compound 1 in a third good solvent and adding a third anti-solvent while stirring to give crystalline form 6 of compound 1; or
the method comprises: dissolving compound 1 in an eighth solvent at 10-30 °C and standing for evaporation to give crystalline form 6 of compound 1.

In another preferred embodiment, the fifth solvent is selected from: ethanol, a combination of acetone and water, a combination of tetrahydrofuran and methyl *tert*-butyl ether, or a combination of anisole and *n*-propanol.

In another preferred embodiment, the sixth solvent is selected from: n-butanol, ethanol, cyclopentyl methyl ether, a combination of isopropyl acetate and *n*-propanol, a combination of tetrahydrofuran and water, a combination of methyl isobutyl ketone and *n*-hexane, a combination of methanol and methyl *tert*-butyl ether, a combination of 2-methyltetrahydrofuran and *n*-heptane, a combination of acetone and water, a combination of anisole and cumene, or a combination of dichloromethane and methyl *tert*-butyl ether.

In another preferred embodiment, the seventh solvent is selected from: methanol, ethanol, acetone, tetrahydrofuran, 1,4-dioxane, or cyclopentyl methyl ether.

In another preferred embodiment, the third good solvent is methanol or acetone; the third anti-solvent is *n*-heptane or water.

In another preferred embodiment, the eighth solvent is selected from: methanol, acetone, or a combination thereof.

In another aspect, the present invention provides a method for preparing crystalline form 7 of compound 1, comprising: in a nitrogen atmosphere, heating crystalline form 6 of compound 1 to 150±20 °C to give crystalline form 7 of compound 1.

In another aspect, the present invention provides a method for preparing crystalline form 8 of compound 1, comprising: dissolving compound 1 in a fourth good solvent and adding a fourth anti-solvent while stirring to give crystalline form 8 of compound 1.

In another preferred embodiment, the fourth good solvent is dimethylformamide; the fourth anti-solvent is water.

In another aspect, the present invention provides a method for preparing crystalline form 9 of compound 1, wherein
the method comprises: stirring compound 1 in a ninth solvent in a temperature cycle to give crystalline form 9 of compound 1, the temperature cycle comprising stirring at 50 °C (e.g., for 1-3 h), cooling to 5 °C and stirring (e.g., for 0.1-1 h), and heating to 50 °C and stirring (e.g., for 0.1-1 h); or
the method comprises: suspending and stirring compound 1 in a ninth solvent at 10-30 °C to give crystalline form 9 of compound 1.

In another preferred embodiment, the ninth solvent is water.

In another aspect, the present invention provides a method for preparing crystalline form 10 of compound 1, comprising: incubating crystalline form 9 of compound 1 in a nitrogen atmosphere at 30±5 °C to give crystalline form 10 of compound 1.

In another aspect, the present invention provides a method for preparing crystalline form 11 of compound 1, wherein
the method comprises: suspending and stirring compound 1 in a tenth solvent at 10-30 °C to give crystalline form 11 of compound 1; or
the method comprises: stirring compound 1 in a tenth solvent in a temperature cycle to give crystalline form 11 of compound 1, the temperature cycle comprising stirring at 50 °C (e.g., for 1-3 h), cooling to 5 °C and stirring (e.g., for 0.1-1 h), and heating to 50 °C and stirring (e.g., for 0.1-1 h).

In another preferred embodiment, the tenth organic solvent is a combination of acetonitrile and toluene.

In another aspect, the present invention provides a method for preparing crystalline form 12 of compound 1, wherein
the method comprises: dissolving compound 1 in a fifth good solvent and subjecting to gas-liquid diffusion in a fifth anti-solvent atmosphere to give crystalline form 12 of compound 1; or
the method comprises: dissolving compound 1 in an eleventh solvent and standing for evaporation to give crystalline form 12 of compound 1; or
the method comprises: subjecting compound 1 to gas-solid diffusion in a twelfth solvent atmosphere to give crystalline form 12 of compound 1.

In another preferred embodiment, the fifth good solvent is selected from: acetone or isopropyl acetate; the fifth anti-solvent is selected from: toluene or methyl tert-butyl ether.

In another preferred embodiment, the eleventh solvent is ethyl acetate.

In another preferred embodiment, the twelfth solvent is methyl ethyl ketone.

In another aspect, the present invention provides a method for preparing crystalline form 13 of compound 1, comprising: incubating crystalline form 12 of compound 1 in a nitrogen atmosphere at 10-30 °C to give crystalline form 13 of compound 1.

The major advantages of the present invention are as follows: Through intensive research, the inventor found a series of polymorphs with good stability and low hydroscopicity. Such polymorphs have good physical and chemical stability, and are suitable for use in pharmaceutic preparations and industrial production; in some preferred embodiments, the crystalline forms of the present invention have more favorable pharmacodynamics and pharmacokinetics and bioavailability, and can be further developed into clinical medicaments for preventing and treating diseases associated with KRAS G12C mutation.

It will be appreciated that within the scope of the present invention, the various technical features of the present invention described above and those specifically described below (e.g., in the examples) may be combined with each other to form new or preferred embodiments. Due to the limited space, such embodiments are not detailed herein.

### BRIEF DESCRIPTION OF THE DRAWINGS

FIG. 1 is an XRPD pattern of crystalline form 1 prepared in Example 1.
FIG. 2 is TGA/DSC patterns of crystalline form 1 prepared in Example 1.
FIG. 3 is a ¹H NMR spectrum of crystalline form 1 prepared in Example 1.
FIG. 4 is an HPLC profile of crystalline form 1 prepared in Example 1.
FIG. 5 is an XRPD pattern of crystalline form 2 prepared in Example 2.
FIG. 6 is TGA/DSC patterns of crystalline form 2 prepared in Example 2.
FIG. 7 is a ¹H NMR spectrum of crystalline form 2 prepared in Example 2.
FIG. 8 is an HPLC profile of crystalline form 2 prepared in Example 2.
FIG. 9 is an XRPD pattern of crystalline form 3 prepared in Example 3.
FIG. 10 is TGA/DSC patterns of crystalline form 3 prepared in Example 3.
FIG. 11 is a ¹H NMR spectrum of crystalline form 3 prepared in Example 3.
FIG. 12 is an XRPD pattern of crystalline form 4 prepared in Example 9.
FIG. 13 is TGA/DSC patterns of crystalline form 4 prepared in Example 9.
FIG. 14 is a ¹H NMR spectrum of crystalline form 4 prepared in Example 9.
FIG. 15 is an XRPD pattern of crystalline form 5 prepared in Example 11.
FIG. 16 is TGA/DSC patterns of crystalline form 5 prepared in Example 11.
FIG. 17 is an XRPD pattern of crystalline form 6 prepared in Example 12.
FIG. 18 is TGA/DSC patterns of crystalline form 6 prepared in Example 12.
FIG. 19 is a ¹H NMR spectrum of crystalline form 6 prepared in Example 12.
FIG. 20 is an XRPD pattern of crystalline form 7 prepared in Example 38.
FIG. 21 is an XRPD pattern of crystalline form 8 prepared in Example 40.
FIG. 22 is TGA/DSC patterns of crystalline form 8 prepared in Example 40.
FIG. 23 is a ¹H NMR spectrum of crystalline form 8 prepared in Example 40.
FIG. 24 is an XRPD pattern of crystalline form 9 prepared in Example 42.
FIG. 25 is TGA/DSC patterns of crystalline form 9 prepared in Example 42.
FIG. 26 is a ¹H NMR spectrum of crystalline form 9 prepared in Example 42.
FIG. 27 is an XRPD pattern of crystalline form 10 prepared in Example 44.
FIG. 28 is an XRPD pattern of crystalline form 11 prepared in Example 46.
FIG. 29 is TGA/DSC patterns of crystalline form 11 prepared in Example 46.
FIG. 30 is a ¹H NMR spectrum of crystalline form 11 prepared in Example 46.
FIG. 31 is an XRPD pattern of crystalline form 12 prepared in Example 49.
FIG. 32 is TGA/DSC patterns of crystalline form 12 prepared in Example 49.
FIG. 33 is a ¹H NMR spectrum of crystalline form 12 prepared in Example 49.
FIG. 34 is an XRPD pattern of crystalline form 13 prepared in Example 53.
FIG. 35 is a DVS pattern of crystalline form 6 in Test Example 2.
FIG. 36 is a DVS pattern of crystalline form 3 in Test Example 2.
FIG. 37 is a DVS pattern of the amorphous form sample in Test Example 2.
FIG. 38 is an XRPD pattern for Compound 1 prepared in Preparative Example 1.

In the TGA/DSC patterns of all the crystalline forms herein, the upper curve is the TGA pattern and the lower curve is the DSC pattern.

### DETAILED DESCRIPTION

The compound of the present invention is compound 1 with the structure of

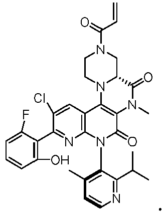

Compound 1 is one of the atropisomers of compound 2, and is named (4aR,8R)-3-acryloyl-11-chloro-10-(2-fluoro-6-hydroxyphenyl)-8-(2-isopropyl-4-methylpyridin-3-yl)-6-methyl-2,3,4,4a,6,8-hexahydro-1H-pyrazino[1',2':4,5]pyrazino[2,3-c][1,8]naphthyridine-5,7-dione. The absolute configuration of the atropisomer is determined by single-crystal X-ray diffraction. As used herein, "compound 1" and "free compound 1" are used interchangeably. The preparation of the compound of the present invention may be implemented with reference to the procedures of the preparative examples herein or to the procedures of Example 25 in International Patent Application No. PCT/CN2020/124226, which is incorporated herein by reference in its entirety. The pharmaceutically acceptable salt of the compound of the present invention is a maleate, fumarate, citrate, p-toluenesulfonate, methanesulfonate, hydrochloride, or phosphate of the compound.

The solid is present either in an amorphous form or a crystalline form. In the case of crystalline forms, the molecules are positioned within a three-dimensional lattice. When a compound crystallizes from a solution or slurry, it may crystallize in different spatial lattice arrangements (known as "polymorphism") to give crystals in different crystalline forms. Such crystalline forms are referred to as "polymorphs". The "crystallization" may be achieved by a number of methods, in particular with reference to the procedures described in the examples of the present invention or to Crystallization, third edition, J W Mullens, Butterworth-Heineman Ltd., 1993, ISBN 0750611294. In particular, the optimization of crystallization may include seeding a crystal of the desired form in a crystallization medium.

The polymorph of the present invention includes a polymorph of the compound of the present invention or a pharmaceutically acceptable salt thereof. In the polymorph of the present invention, the pharmaceutically acceptable salt is a hydrochloride or phosphate. Herein, the terms "crystal of the present invention", "crystalline form of the present invention", "polymorph of the present invention", and the like are used interchangeably.

The active ingredient of the present invention may be the pharmaceutically acceptable salt of the compound of the present invention or the polymorph of the present invention. The active ingredients of the present invention may be used to inhibit the activity of KRAS G12C mutation. Therefore, the active ingredient of the present invention and a pharmaceutical composition comprising the active ingredient of the present invention may be used to treat or prevent a disease associated with KRAS G12C mutation, such as a cancer associated with KRAS G12C mutation. The cancer may be a solid tumor or a hematological tumor. For example, the cancer includes (but is not limited to) one or more selected from: lung cancer (e.g., non-small cell lung cancer), pancreatic cancer, colorectal cancer, and the like.

The pharmaceutical composition of the present invention comprises the active ingredient of the present invention and a pharmaceutically acceptable carrier. The pharmaceutical composition of the present invention may also comprise, optionally, an additional therapeutic agent. As used herein, the "pharmaceutically acceptable carrier" refers to a non-toxic, inert, solid or semi-solid substance, a liquid filler, diluent, encapsulating material or auxiliary agent, or any excipient that is compatible with the patient, preferably a mammal, more preferably a human, and suitable for delivering an active agent to a target of interest without terminating the activity of the agent. The agent of the present invention may be used alone or in combination with one or more additional therapeutic agents as desired during the course of treatment. The combination may refer to the concurrent administration of the agent of the present invention and one or more additional therapeutic agents, or the administration of one or more additional therapeutic agents prior to or after the administration of the agent of the present invention.

In general, the active ingredient of the present invention may be administered in suitable dosage forms formulated with one or more pharmaceutically acceptable carriers. Such dosage forms are suitable for oral, rectal, topical, intraoral, and other parenteral (e.g., subcutaneous, intramuscular, intravenous, etc.) administration. For example, suitable dosage forms for other parenteral administration include injections and the like. The aforementioned dosage forms can be prepared from the active ingredient of the present invention and one or more carriers or excipients by conventional pharmaceutical methods. The aforementioned carrier needs to be compatible with the active ingredient of the present invention or other excipients. For solid formulations, common non-toxic carriers include, but are not limited to, mannitol, lactose, starch, magnesium stearate, glucose, sucrose, and the like. The carrier for liquid formulations includes water (preferably sterile water for injection) and the like. The active ingredient of the present invention may be combined with the aforementioned carriers to form a solution or a suspension.

The pharmaceutical composition of the present invention is formulated, quantified, and administered as per pharmaceutical practices. The "therapeutically effective amount" for administering the active ingredient of the present invention will be determined by such factors as the particular disorder of interest, the individual of interest, the cause of the disorder, the target of the agent, and the mode of administration. As used herein, the "therapeutically effective amount" refers to an amount that produces a function or activity in a patient (e.g., a human and/or an animal) and is acceptable in humans and/or animals. The therapeutically effective amount of the pharmaceutical composition of the present invention or the active ingredient contained in the pharmaceutical composition is preferably 0.1 mg/kg to 5 g/kg (body weight). In general, for dosages used for the treatment of adults, the dosage will generally be in the range of 0.02-5000 mg/day, for example, about 1-1500 mg/day. The dose may be presented as a single dose, or doses for concurrent administration, or sub-doses at appropriate intervals, for example, two, three, four, or more sub-doses per day. It will be appreciated by those skilled in the art that, although the above dosage ranges are given, the specific effective amount may be adjusted as appropriate to the patient's condition in combination with the physician's diagnosis.

As used herein, the "patient" refers to an animal, preferably a mammal, more preferably a human. The term "mammal" refers to warm-blooded vertebrate mammals, including, for example, cats, dogs, rabbits, bears, foxes, wolves, monkeys, deer, mice, pigs, and humans.

As used herein, "treating" or "treatment" refers to the alleviation, delayed progression, attenuation, prevention, or maintenance of an existing disease or disorder (e.g., cancer). The "treatment" also includes the curing, prevention of development, or alleviation to some extent of one or more symptoms of a disease or disorder.

As the active ingredient of the present invention, the pharmaceutically acceptable salt of compound 1, or a series of stable polymorphs of the compound or the pharmaceutically acceptable salt thereof, may be prepared by a variety of synthetic methods well known to those skilled in the art, including the exemplified examples below, embodiments formed by combinations with other chemical synthetic methods, and equivalents thereof well known to those skilled in the art, with preferred embodiments including but not limited to the examples of the present invention.

The present invention will be further illustrated with reference to the following specific examples. It will be appreciated that such examples are merely intended to illustrate the present invention rather than limit the scope of the present invention. Procedures without specified conditions in the following examples are generally conducted in conventional conditions, or conditions recommended by the manufacturers.

Unless otherwise indicated, percentages and parts are on weight basis. Unless otherwise defined, terms used herein have the same meaning as are familiar to those skilled in the art. Unless otherwise defined, any reagent or apparatus used herein is commercially available. Any procedures and materials similar or equivalent to those described herein can be used for implementing the present invention. As used herein, the term "room temperature" includes temperatures of 4-30 °C, and generally refers to 15-30 °C.

The polymorph prepared according to the present invention may be characterized by a variety of means and instruments, such as XRPD, DSC, TGA, IR, and the like. The polymorph of the present invention has a specific crystalline form and has specific characteristic peaks in an XRPD pattern.

The X-ray powder diffraction (XRPD) analysis may be conducted on the X' Pert3 X-ray powder diffraction analyzer with the following parameters: Cu, k•; K•1 (Å): 1.540598; K•2 (Å): 1.544426; K•2/K•1 intensity ratio: 0.50; voltage: 45 kV; current: 40 mA; divergent slit: 1/8°; scan mode: continuous; scanning range: 3° to 40°; scanning step: 0.0263°; time of step: 46.665 s; scanning time: 5 min 03 s. Alternatively, the analysis may be conducted on the PANalytical Empyrean X-ray powder diffraction analyzer with the following parameters: Cu, k•; K•1 (Å): 1.540598; K•2 (Å): 1.544426; K•2/K• 1 intensity ratio: 0.50; voltage: 45 kV; current: 40 mA; divergent slit: automatic; scan mode: continuous; scanning range: 3° to 40°; scanning step: 0.0167°; time of step: 17.780 s/33.020 s; scanning time: 5 min 32 s/10 min 13 s.

The thermogravimetric analysis (TGA) may be conducted on the TA Q5000/Discovery 5500 thermogravimetric analyzer. Mode: linear heating; sample tray: aluminum tray, open; temperature range: RT-350 °C; ramping rate: 10 °C/min; protective gas: nitrogen.

The differential scanning calorimetry (DSC) analysis may be conducted on the TA Discovery 2500 differential scanning calorimeter. Mode: linear heating; sample tray: aluminum tray, capped; temperature range: RT to target temperature; ramping rate: 10 °C/min; protective gas: nitrogen.

The dynamic vapor sorption (DVS) analysis may be conducted on the DVS Intrinsic system from Surface Measurement Systems (SMS). Temperature: 25 °C; sample amount: 20-40 mg; protective gas and flow rate: N₂, 200 mL/min; dm/dt: 0.002%/min; minimum dm/dt equilibration time: 10 min; maximum equilibration time: 180 min; RH range: room humidity to 95% RH to 0% RH to 95% RH; 0% RH to 95% RH to 0% RH; RH gradient: 10% RH (0% RH to 90% RH & 90% RH to 0% RH); 5% RH (90% RH to 95% RH & 95% RH to 90% RH).

Proton nuclear magnetic resonance may be conducted on the Bruker 400M nuclear magnetic resonance spectrometer, with deuterated chloroform, deuterated methanol, or dimethyl sulfoxide-d6 as the solvent.

Water titration (KF test) may be conducted on the Metrohm 870 KF Titrinoplus from Sigma-Aldrich, with ultrapure water for calibration and Hydranal^{®} R-Composite 5 as the titration reagent. HPLC-grade methanol was used to dissolve the solid sample.

High-performance liquid chromatography (HPLC) may be conducted on the Agilent 1260 high-performance liquid chromatograph system (Waters Xbridge C18, 150 × 4.6 mm, 5 µm column). Ion chromatography (IC) may be conducted on the ThermoFisher ICS-1100 ion chromatograph system (IonPac AS18 Analytical Column (4 × 250 mm)).

It will be appreciated that other similar instruments to those described above or test conditions different from those used in the present invention may give other values, and therefore the values quoted herein should not be construed as absolute values. As will be appreciated by those skilled in the art due to instrumental errors or operator differences, the above parameters used to characterize the physical properties of the crystals may vary slightly and are therefore used only for reference in characterizing the polymorphs provided herein, and should not be construed as limiting the polymorphs of the present invention.

### Preparative Example 1. Preparation of compound 1

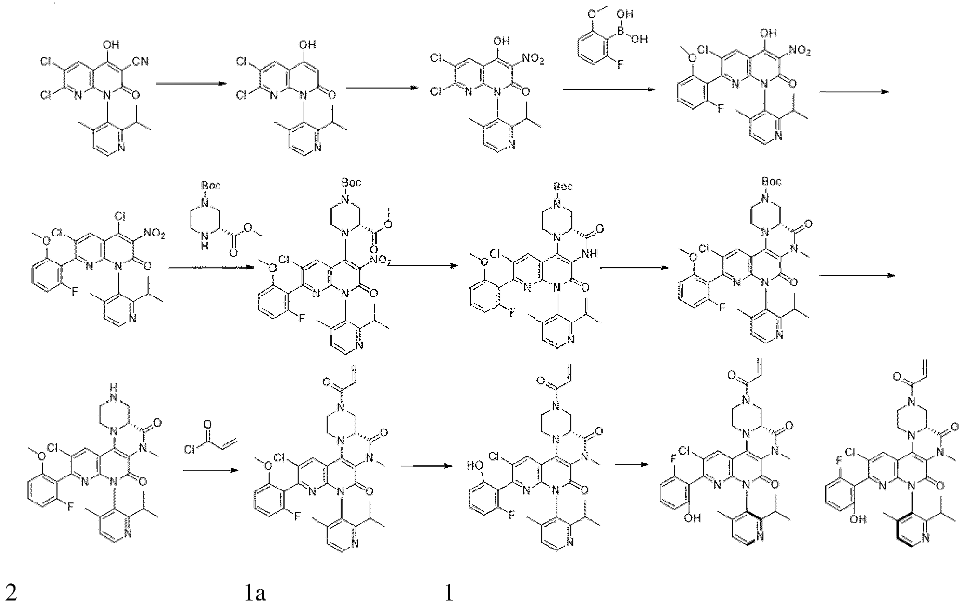

Step 1: 6,7-Dichloro-4-hydroxy-1-(2-isopropyl-4-methylpyridin-3-yl)-2-oxo-1,2-dihydro-1,8-naphthyridine-3-carbonitrile (30.0 g, 77.319 mmol) was suspended in a mixed solution of 1,4-dioxane (120 mL) and water (120 mL), before concentrated sulfuric acid (120 mL) was slowly added. The reaction system was stirred at 120 °C for 36 hours. The reaction solution was cooled, poured into 200 mL of ice water, adjusted to pH 2-3 with sodium carbonate, and extracted with ethyl acetate (1000 mL × 2). The organic phases were combined, dried over anhydrous sodium sulfate, and filtered. The filtrate was concentrated in vacuum to give a pale brown solid product 6,7-dichloro-4-hydroxy-1-(2-isopropyl-4-methylpyridin-3-yl)-1,8-naphthyridin-2(1H)-one (24 g, 85.7% yield). ES-API: [M+H]⁺=364.1.

Step 2: 6,7-Dichloro-4-hydroxy-1-(2-isopropyl-4-methylpyridin-3-yl)-1,8-naphthyridin-2(1H)-one (3.16 g, 8.705 mmol) was dissolved in acetic acid (15 mL), before sodium nitrite (100 mg, 1.58 mmol) and concentrated nitric acid (5.0 mL, 74.52 mmol) were sequentially added. The reaction system was stirred at room temperature for 30 minutes. The reaction solution was slowly poured into 100 mL of ice water. The precipitate was collected by filtration and was washed with 20 mL of ice water. The filtrate was concentrated in vacuum to give a yellow solid product 6,7-dichloro-4-hydroxy-1-(2-isopropyl-4-methylpyridin-3-yl)-3-nitro-1,8-naphthyridin-2(1H)-one (3.5 g, 92% yield). ES-API: [M+H]⁺=409.1.

Step 3: 6,7-Dichloro-4-hydroxy-1-(2-isopropyl-4-methylpyridin-3-yl)-3-nitro-1,8-naphthyridin-2(1H)-one (3.5 g, 8.570 mmol), (2-fluoro-6-methoxyphenyl)boronic acid (5.8 g, 34.10 mmol), tetrakis(triphenylphosphine)palladium(0) (1.15 g, 0.9956 mmol), sodium carbonate (3.5 g, 33.02 mmol), 10 mL of water, and 40 mL of dioxane were added into a 100-mL three-necked flask. In a nitrogen atmosphere, the system was stirred for 2-3 hours at 100 °C for reaction. The reaction solution was cooled to room temperature, before 80 mL of water and 100 mL of methyl tert-butyl ether were added. The mixture was extracted once. The aqueous phase was adjusted to pH 3-5 by adding 1 M hydrochloric acid and extracted with ethyl acetate (200 mL × 2). The organic phases were combined, dried over anhydrous sodium sulfate, and filtered. The filtrate was concentrated in vacuum to give a pale yellow solid product 6-chloro-7-(2-fluoro-6-methoxyphenyl)-4-hydroxy-1-(2-isopropyl-4-methylpyridin-3-yl)-3-nitro-1,8-naphthyridin-2(1H)-one (4.5 g, crude). ES-API: [M+H]⁺=499.1.

Step 4: 6-Chloro-7-(2-fluoro-6-methoxyphenyl)-4-hydroxy-1-(2-isopropyl-4-methylpyridin-3-yl)-3-nitro-1,8-naphthyridin-2(1H)-one (4.6 g, 8.57 mmol) was dissolved in acetonitrile (30 mL), before phosphorus oxychloride (7.5 g, 48.92 mmol) and N,N-diisopropylethylamine (10.5 g, 81.24 mmol) were sequentially added. The reaction system was heated to 80 °C and stirred for 30 min. The reaction solution was concentrated before 30 mL of cold acetonitrile was added. In an ice-water bath, the mixture was dropwise added to 150 mL of saturated sodium bicarbonate solution, and extracted with ethyl acetate (200 mL × 2). The organic phases were combined and washed once with 200 mL of saturated brine. The mixture was dried over anhydrous sodium sulfate, filtered, and concentrated. The crude product was purified on flash silica gel column (ethyl acetate/petroleum ether: 0-50%) to give a yellow solid 4,6-dichloro-7-(2-fluoro-6-methoxyphenyl)-1-(2-isopropyl-4-methylpyridin-3-yl)-3-nitro-1,8-naphthyridin-2(1H)-one (3.05 g, 76% yield). ES-API: [M+H]⁺=517.2.

Step 5: 4,6-Dichloro-7-(2-fluoro-6-methoxyphenyl)-1-(2-isopropyl-4-methylpyridin-3-yl)-3-nitro-1,8-naphthyridin-2(1H)-one (2.5 g, 4.843 mmol) was dissolved in N,N-dimethylacetamide (25 mL), before 1-(*tert*-butyl) 3-methyl(R)-piperazine-1,3-dicarboxylate (3.5 g, 14.34 mmol) and N,N-diisopropylethylamine (2.0 g, 15.47 mmol) were sequentially added. The reaction system was stirred at 120 °C for 2 hours. 80 mL of ethyl acetate was added to the reaction solution, and the mixture was washed with 80 mL of saturated brine thrice. The organic phase was dried and concentrated, and the crude product was purified on a flash silica gel column (ethyl acetate/petroleum ether: 0-80%) to give a yellow solid product 1-(*tert*-butyl) 3-methyl(3R)-4-(6-chloro-7-(2-fluoro-6-methoxyphenyl)-1-(2-isopropyl-4-methylpyridin-3-yl)-3-nitro-2-oxo-1,2-dihydro-1,8-naphthyridin-4-yl)piperazine-1,3-dicarboxylate (2.7 g, 77% yield). ES-API: [M+H]⁺=725.2.

Step 6: 1-(*tert*-Butyl) 3-methyl(3R)-4-(6-chloro-7-(2-fluoro-6-methoxyphenyl)-1-(2-isopropyl-4-methylpyridin-3-yl)-3-nitro-2-oxo-1,2-dihydro-1,8-naphthyridin-4-yl)piperazine-1,3-dicarboxylate (2.7 g, 3.728 mmol) was dissolved in acetic acid (30 mL) before iron powder (835 mg, 14.91 mmol) was added. The reaction system was stirred at 80 °C for 30 min. The reaction solution was concentrated before 200 mL of ethyl acetate and 100 mL of saturated sodium bicarbonate were sequentially added. The suspension was filtered through celite, and the filter cake was washed with ethyl acetate. The organic phase was separated, sequentially washed with 100 mL of saturated sodium bicarbonate and 150 mL of saturated brine, dried, and concentrated to give a yellow solid product *tert*-butyl (4aR)-11-chloro-10-(2-fluoro-6-methoxyphenyl)-8-(2-isopropyl-4-methylpyridin-3-yl)-5,7-dioxo-1,2,4,4a,5,6,7,8-octahydro-3H-pyrazino[1',2':4,5]pyrazino[2,3-c][1,8]naphthyridine-3-carboxylate (2.70 g, crude). ES-API: [M+H]⁺=663.2.

Step 7: *tert*-Butyl (4aR)-11-chloro-10-(2-fluoro-6-methoxyphenyl)-8-(2-isopropyl-4-methylpyridin-3-yl)-5,7-dioxo-1,2,4,4a,5,6,7,8-octahydro-3H-pyrazino[1',2':4,5]pyrazino[2,3-c][1,8]naphthyridine-3-carboxylate (2.7 g, 3.728 mmol), 30 mL of acetone, anhydrous potassium carbonate (2.2 g, 15.94 mmol), and iodomethane (5.4 g, 38.03 mmol) were sequentially added to a 150-mL tube. After the tube was sealed, the reaction system was stirred at 55 °C for 18 hours. 150 mL of ethyl acetate was added to the reaction solution, and the mixture was washed with 100 mL of saturated brine thrice, dried, and concentrated. The crude product was purified on a flash silica gel column (ethyl acetate/petroleum ether: 0-80%) to give a yellow solid product *tert*-butyl (4aR)-11-chloro-10-(2-fluoro-6-methoxyphenyl)-8-(2-isopropyl-4-methylpyridin-3-yl)-6-methyl-5,7-dioxo-1,2,4,4a,5,6,7,8-octahydro-3H-pyrazino[1',2':4,5]pyrazino[2,3-c] [1,8]naphthyridine-3-carboxylate (2.2 g, 87% yield). ES-API: [M+H]⁺=677.2.

Step 8: *tert*-Butyl (4aR)-11-chloro-10-(2-fluoro-6-methoxyphenyl)-8-(2-isopropyl-4-methylpyridin-3-yl)-6-methyl-5,7-dioxo-1,2,4,4a,5,6,7,8-octahydro-3H-pyrazino[1',2':4,5]pyrazino[2,3-c][1,8]naphthyridine-3-carboxylate (517 mg,0.7549 mmol) was dissolved in dichloromethane (8 mL) before trifluoroacetic acid (2 mL) was added. The reaction solution was stirred at room temperature for 2 hours and concentrated to give a product (4aR)-11-chloro-10-(2-fluoro-6-methoxyphenyl)-8-(2-isopropyl-4-methylpyridin-3-yl)-6-methyl-2,3,4,4a,6,8-hexahydro-1H-pyrazino[1',2':4,5]pyrazino[2,3-c][1,8]naphthyridine-5,7-dione (530 mg, crude), which was directly used in the next reaction. ES-API: [M+H]⁺=577.2.

Step 9: (4aR)-11-Chloro-10-(2-fluoro-6-methoxyphenyl)-8-(2-isopropyl-4-methylpyridin-3-yl)-6-methyl-2,3,4,4a,6,8-hexahydro-1H-pyrazino[1',2':4,5]pyrazino[2,3-c][1,8]naphthyridine-5,7-dione (530 mg, 0.7549 mmol) was dissolved in dichloromethane (15 mL) before triethylamine (3.0 mL, 21.62 mmol) was added. The reaction solution was cooled to 0 °C before acryloyl chloride (100 mg, 1.1048 mmol) was dropwise added. The reaction system was stirred at 0 °C for 15 min. 80 mL of dichloromethane was added to the reaction solution. The mixture was washed with 100 mL of saturated aqueous NaHCO₃ solution and 80 mL of saturated brine, dried, and concentrated. The crude product was purified on a flash silica gel column (ethyl acetate/petroleum ether: 0-60%) to give a yellow solid product (4aR)-3-acryloyl-11-chloro-10-(2-fluoro-6-methoxyphenyl)-8-(2-isopropyl-4-methylpyridin-3-yl)-6-methyl-2,3,4,4a,6,8-hexahydro-1H-pyrazino[1',2':4,5]pyrazino[2,3-c][1,8]naphthyridine-5,7-dione (280 mg, 59% yield). ES-API: [M+H]⁺=631.2.

Step 10: In an ice-water bath, (4aR)-3-acryloyl-11-chloro-10-(2-fluoro-6-methoxyphenyl)-8-(2-isopropyl-4-methylpyridin-3-yl)-6-methyl-2,3,4,4a,6,8-hexahydro-1H-pyrazino[1',2':4,5]pyrazino[2,3-c][1,8]naphthyridine-5,7-dione (280 mg, 0.444 mmol) was added to anhydrous dichloromethane (6.0 mL) before boron tribromide (5.0 mL, 5.0 mmol) was added. The reaction system was warmed to room temperature and incubated overnight. In an ice-water bath, the reaction solution was dropwise added into saturated sodium bicarbonate solution. The mixture was extracted with dichloromethane (80 mL) twice, dried, and concentrated. The crude product was purified on a flash silica gel column (ethyl acetate/petroleum ether: 0-60%) to give a product (4aR)-3-acryloyl-11-chloro-10-(2-fluoro-6-hydroxyphenyl)-8-(2-isopropyl-4-methylpyridin-3-yl)-6-methyl-2,3,4,4a,6,8-hexahydro-1H-pyrazino[1',2':4,5]pyrazino[2,3-c][1,8]naphthyridine-5,7-dione (compound 2, a mixture of compound 1a and compound 1, 233 mg, 85% yield). ¹H NMR (400 MHz, CDC1₃): • 8.58 - 8.52(m, 1H), 8.35(s, 1H), 8.32(brs, 1H), 7.26 - 7.19(m, 2H), 7.13 - 7.05(m, 1H), 6.71 - 6.74(m, 2H), 6.41 - 6.36(dd, J1 = 16.8 Hz, J2 = 2.0 Hz, 1H), 5.84(d, J = 11.2 Hz, 1H), 4.93 - 4.81(m, 2H), 3.68(m, 2H), 3.52(s, 1.5H), 3.50(s, 1.5H), 3.45 - 3.39(m, 1H), 3.19 - 3.12(m, 1H), 2.78 - 2.72(m, 0.5H), 2.44 - 2.41(m, 0.5H), 2.12(s, 1.5H), 1.92(s, 1.5H), 1.24(d, J = 6.8 Hz, 1.5H), 1.18(d, J = 6.8 Hz, 1.5H), 1.11(d, J = 6.8 Hz, 1.5H), 1.00(d, J = 6.8 Hz, 1.5H).

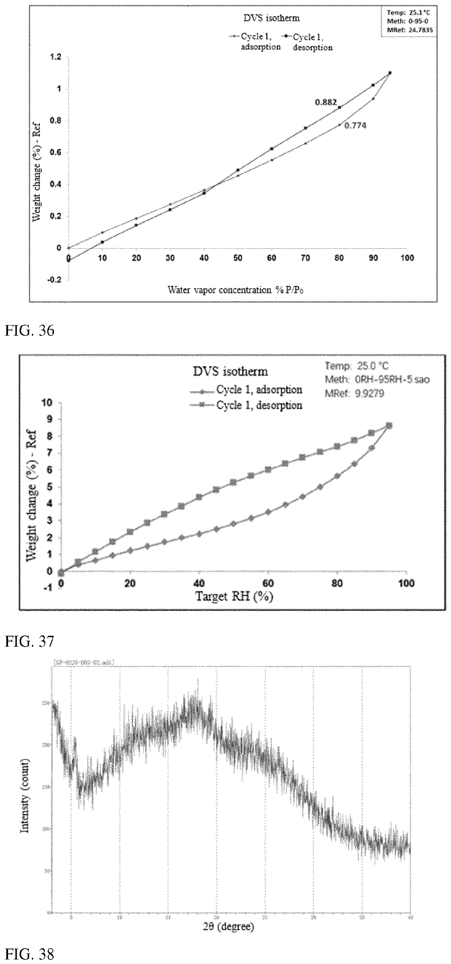

Step 11: Compound 2 obtained in the above step was separated by preparative chiral HPLC (column: IA: 10•m, 30 × 250 mm; mobile phase: hexane:ethanol = 60:40; flow rate: 25 mL/min; column temperature: room temperature) to give compound 1 (70 mg, peak 2, retention time 3.683 min, 31% yield). ¹H NMR (500 MHz, CDCl₃) • 8.64-8.59 (m, 1H), 8.35 (s, 1H), 8.07 (s, 1H), 7.27-7.20 (m, 2H), 7.14-7.02 (m, 1H), 6.75-6.63 (m, 2H), 6.39 (dd, *J* = 17.0, 2.0 Hz, 1H), 5.88-5.77 (m, 1H), 4.91 (d, *J* = 14.0 Hz, 1H), 4.83 (d, *J* = 13.0 Hz, 1H), 3.72-3.58 (m, 2H), 3.50 (s, 3H), 3.43 (d, *J* = 12.0 Hz, 1H), 3.16 (t, *J* = 13.0 Hz, 1H), 2.91 (t, *J* = 12.0 Hz, 1H), 2.82-2.73 (m, 1H), 1.93 (s, 3H), 1.24 (d, *J* = 7.0 Hz, 3H), 1.12 (d, *J* = 7.0 Hz, 3H). ES-API: [M+H]⁺=617.2. Compound 1 was detected by analytical chiral HPLC method (column: IA: 5 • m, 4.6 × 150 mm, mobile phase: hexane:ethanol = 60:40, flow rate: 1 mL/min, column temperature = 30 °C). Compound 1 was subjected XPRD test, and the results are shown in FIG. 38. The test confirmed that it was an amorphous form (compound 1 was subjected to single-crystal growth; see Test Example 7 in PCT/CN2020/124226).

The compound 1 obtained in Preparative Example 1 was used as the starting material for the screening and preparation of salt forms or crystalline forms in the following examples.

The temperature cycle (50 °C to 5 °C) method used in the following examples comprises the following steps: stirring at 50 °C for 2 hours; cooling to 5 °C and stirring for 0.5 hours at 5 °C; heating to 50 °C and stirring for 0.5 hours at 50 °C; and repeating the procedures once, cooling to 5 °C, and holding at 5 °C.

The anti-solvent addition in the following examples comprises the following steps: dissolving 15 mg of Compound 1 prepared in Preparative Example 1 in 0.1-0.8 mL of the corresponding good solvent, and dropwise adding the corresponding anti-solvent the solution while stirring until a solid is precipitated.

The gas-solid diffusion in the following examples comprises the following steps: placing the solid compound 1 prepared in Preparative Example 1 in an atmosphere of different solvents in a closed environment, and letting stand at room temperature for about 10 days for gas-solid diffusion. The gas-liquid diffusion in the following examples comprises the following steps: dissolving 15 mg of compound 1 obtained in Preparative Example 1 in 0.2-0.8 mL of different solvents, and letting the solution stand in an atmosphere of different anti-solvents at room temperature in a closed environment for gas-liquid diffusion.

### Example 1. Preparation of crystalline form 1 of compound 1 hydrochloride

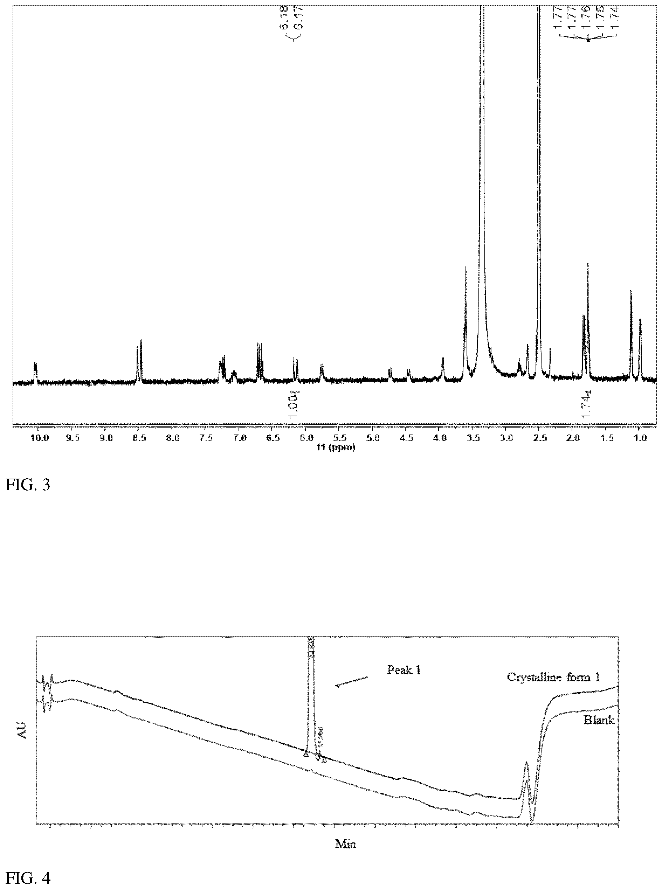

Compound 1 (15 mg) prepared in Preparative Example 1 and hydrochloric acid were added in a 1:2 molar ratio to 1 mL of tetrahydrofuran and the mixture was stirred at room temperature for 3 days. The solid was separated at room temperature and dried in vacuum for 4 hours to give crystalline form 1 of compound 1 hydrochloride. The XRPD results are shown in FIG. 1 and Table 1. The TGA/DSC results are shown in FIG. 2. The TGA results showed a weight loss of 2.7% at 100 °C and of 15.7% from 100 °C to 250 °C. The DSC results showed one endothermic peak at 62.0 °C and one endothermic peak at 238.0 °C. The ¹H NMR results, as shown in FIG. 3, indicated a 0.05 wt% tetrahydrofuran residue in crystalline form 1. The HPLC results, as shown in FIG. 4 and Table 2, indicated a 99.93% HPLC purity. The IC results showed a 0.9:1 molar ratio of the ligand acid to free compound 1.

**Table 1. Crystalline form 1**

| Pos. [°2•] | d [Å] | Rel. Int. [%] | Pos. [°2•] | d [Å] | Rel. Int. [%] |
|---|---|---|---|---|---|
| 8.37 | 10.56 | 16.24 | 21.75 | 4.09 | 25.11 |
| 9.98 | 8.86 | 30.79 | 22.34 | 3.98 | 3.68 |
| 10.91 | 8.11 | 7.84 | 23.57 | 3.77 | 19.06 |
| 11.69 | 7.57 | 15.57 | 25.22 | 3.53 | 19.32 |
| 12.99 | 6.81 | 33.22 | 26.79 | 3.33 | 8.19 |
| 13.73 | 6.45 | 100.00 | 27.27 | 3.27 | 18.80 |
| 15.08 | 5.87 | 15.53 | 27.61 | 3.23 | 8.43 |
| 16.07 | 5.51 | 8.95 | 29.07 | 3.07 | 5.37 |
| 16.91 | 5.24 | 3.82 | 30.34 | 2.95 | 6.65 |
| 17.46 | 5.08 | 2.09 | 31.19 | 2.87 | 4.76 |
| 18.40 | 4.82 | 11.28 | 32.44 | 2.76 | 2.74 |
| 18.65 | 4.76 | 28.27 | 33.39 | 2.68 | 1.03 |
| 19.87 | 4.47 | 17.27 | 34.72 | 2.58 | 0.88 |
| 20.21 | 4.39 | 4.27 | 35.86 | 2.50 | 2.09 |
| 20.73 | 4.28 | 21.85 | 38.19 | 2.36 | 1.47 |
| 21.58 | 4.12 | 17.08 | | | |

**Table 2. HPLC purity results for crystalline form 1**

| Peak# | RRT | Area (%) |
|---|---|---|
| 1 | 1.00 | 99.93 |
| 2 | 1.03 | 0.07 |

### Example 2. Preparation of crystalline form 2 of compound 1 phosphate

Compound 1 (15 mg) prepared in Preparative Example 1 and phosphoric acid were added in a 1:2 molar ratio to 1 mL of acetonitrile and the mixture was stirred at room temperature for 3 days. The obtained solid was separated at room temperature and dried in vacuum for 4 hours to give crystalline form 2 of compound 1 phosphate. The XRPD results are shown in FIG. 5 and Table 3. The TGA/DSC results are shown in FIG. 6. The TGA results showed a weight loss of 7.9% at 120 °C and of 7.2% from 120 °C to 220 °C. The DSC results showed three endothermic peaks at 68.2 °C, 196.0 °C, and 242.6 °C. The ¹H NMR results, as shown in FIG. 7, indicated no significant acetonitrile residue in crystalline form 2. The HPLC results, as shown in FIG. 8, indicated a 100.00% HPLC purity. The IC results showed a 2.0:1 molar ratio of the ligand acid to free compound 1.

**Table 3. Crystalline form 2**

| Pos. [°2•] | d [Å] | Rel. Int. [%] | Pos. [°2•] | d [Å] | Rel. Int. [%] |
|---|---|---|---|---|---|
| 7.37 | 12.00 | 17.24 | 19.14 | 4.64 | 38.59 |
| 9.74 | 9.08 | 33.55 | 19.53 | 4.54 | 20.60 |
| 10.94 | 8.09 | 32.94 | 20.54 | 4.32 | 52.02 |
| 11.55 | 7.66 | 31.57 | 21.12 | 4.21 | 14.69 |
| 13.24 | 6.69 | 29.36 | 22.35 | 3.98 | 17.24 |
| 14.20 | 6.24 | 100.00 | 24.23 | 3.67 | 36.31 |
| 15.56 | 5.69 | 35.71 | 24.67 | 3.61 | 19.77 |
| 16.02 | 5.53 | 19.67 | 25.88 | 3.44 | 34.73 |
| 16.91 | 5.24 | 19.28 | 27.44 | 3.25 | 7.52 |
| 18.40 | 4.82 | 48.84 | 29.46 | 3.03 | 7.83 |

### Example 3. Preparation of crystalline form 3 of compound 1

15 mg of compound 1 prepared in Preparative Example 1 was suspended in 0.5 mL of methyl *tert-*butyl ether and the mixture was stirred for 4 days at 50 °C. The solid was separated and dried at room temperature in vacuum to give crystalline form 3 of compound 1. The XRPD results are shown in FIG. 9 and Table 4. The TGA/DSC results are shown in FIG. 10. The TGA results showed a weight loss of 1.4% at 200 °C. The DSC results showed one endothermic peak at 292.1 °C. The ¹H NMR results, as shown in FIG. 11, indicated a 0.4 wt% methyl tert-butyl ether residue.

The results of further identification of crystalline form 3 by variable-temperature XRPD showed that no crystalline form changes were observed after heating crystalline form 3 to 150 °C and cooling to 30 °C in a N₂ atmosphere, indicating that crystalline form 3 was an anhydrous crystalline form.

**Table 4. Crystalline form 3**

| Pos. [°2•] | d [Å] | Rel. Int. [%] | Pos. [°2•] | d [Å] | Rel. Int. [%] |
|---|---|---|---|---|---|
| 10.05 | 8.80 | 29.94 | 20.85 | 4.26 | 32.48 |
| 11.38 | 7.78 | 27.90 | 21.53 | 4.13 | 7.08 |
| 11.97 | 7.39 | 24.28 | 22.26 | 3.99 | 2.46 |
| 12.20 | 7.25 | 27.03 | 23.00 | 3.87 | 3.12 |
| 12.58 | 7.04 | 100.00 | 23.74 | 3.75 | 8.10 |
| 13.32 | 6.65 | 18.16 | 24.70 | 3.60 | 13.86 |
| 13.67 | 6.48 | 17.45 | 24.95 | 3.57 | 27.37 |
| 14.47 | 6.12 | 2.24 | 25.29 | 3.52 | 8.19 |
| 15.14 | 5.85 | 5.22 | 25.91 | 3.44 | 7.03 |
| 15.61 | 5.68 | 6.22 | 26.23 | 3.40 | 11.81 |
| 16.34 | 5.43 | 2.72 | 27.52 | 3.24 | 3.80 |
| 17.37 | 5.11 | 26.23 | 27.74 | 3.22 | 4.38 |
| 18.20 | 4.88 | 23.59 | 29.16 | 3.06 | 2.59 |
| 18.56 | 4.78 | 4.06 | 29.94 | 2.98 | 9.98 |
| 18.98 | 4.68 | 18.15 | 30.57 | 2.92 | 12.34 |
| 19.62 | 4.52 | 10.37 | 31.28 | 2.86 | 3.23 |
| 19.73 | 4.50 | 9.19 | 32.47 | 2.76 | 4.18 |
| 20.21 | 4.39 | 13.85 | 35.29 | 2.54 | 1.27 |
| 20.62 | 4.31 | 27.16 | 37.23 | 2.42 | 3.80 |

### Example 4. Preparation of crystalline form 3 of compound 1

15 mg of compound 1 prepared in Preparative Example 1 was suspended in 0.5 mL of n-heptane and the mixture was stirred for 4 days at 50 °C. The obtained solid was dried at room temperature to give a solid product crystalline form 3 of compound 1 as confirmed by XRPD.

### Example 5. Preparation of crystalline form 3 of compound 1

15 mg of compound 1 prepared in Preparative Example 1 was suspended in 0.5 mL of dichloromethane/n-heptane (1:4, v/v) and the mixture was stirred for 4 days at 50 °C. The obtained solid was dried at room temperature to give a solid product crystalline form 3 of compound 1 as confirmed by XRPD.

### Example 6. Preparation of crystalline form 3 of compound 1

15 mg of compound 1 prepared in Preparative Example 1 was suspended in 0.5 mL of isopropyl acetate/n-hexane (1:4, v/v) and the mixture was stirred for 4 days at 50 °C. The obtained solid was dried at room temperature to give a solid product crystalline form 3 of compound 1 as confirmed by XRPD.

### Example 7. Preparation of crystalline form 3 of compound 1

By means of anti-solvent addition (with methyl isobutyl ketone as the good solvent and n-heptane as the anti-solvent), a solid was precipitated, separated by filtration, and dried at room temperature to give a solid product crystalline form 3 of compound 1 as confirmed by XRPD.

### Example 8. Preparation of crystalline form 3 of compound 1

By means of anti-solvent addition (with chloroform as the good solvent and methyl tert-butyl ether as the anti-solvent), a solid was precipitated, separated by filtration, and dried at room temperature to give a solid product crystalline form 3 of compound 1 as confirmed by XRPD.

### Example 9. Preparation of crystalline form 4 of compound 1

15 mg of compound 1 prepared in Preparative Example 1 was subjected to gas-solid diffusion at room temperature in a dimethyl sulfoxide atmosphere for 10 days to give crystalline form 4 of compound 1. The XRPD results are shown in FIG. 12 and Table 5. The TGA/DSC results are shown in FIG. 13. The TGA results showed a weight loss of 5.9% at 150 °C and of 11.3% from 150 °C to 250 °C. The DSC results showed three endothermic peaks at 73.6 °C, 119.7 °C, and 231.1 °C, and two overlapping endothermic peaks at 266.0 °C and 272.8 °C. The ¹H NMR results, as shown in FIG. 14, indicated 12.2 wt% of dimethyl sulfoxide (approximate to the second weight loss in TGA).

The results of further identification of crystalline form 4 by variable-temperature XRPD showed that no significant crystalline form changes were observed when heating crystalline form 4 to 80 °C or 130 °C in a N₂ atmosphere.

**Table 5. Crystalline form 4**

| Pos. [°2•] | d [Å] | Rel. Int. [%] | Pos. [°2•] | d [Å] | Rel. Int. [%] |
|---|---|---|---|---|---|
| 6.00 | 14.74 | 50.01 | 23.12 | 3.85 | 21.20 |
| 6.96 | 12.70 | 30.45 | 23.24 | 3.83 | 25.42 |
| 12.11 | 7.31 | 79.29 | 23.76 | 3.74 | 2.78 |
| 13.04 | 6.79 | 100.00 | 24.33 | 3.66 | 13.11 |
| 13.96 | 6.34 | 23.04 | 24.55 | 3.63 | 28.75 |
| 14.27 | 6.21 | 5.58 | 25.56 | 3.49 | 20.55 |
| 14.71 | 6.02 | 5.87 | 26.01 | 3.43 | 16.53 |
| 15.52 | 5.71 | 2.25 | 26.90 | 3.31 | 22.34 |
| 16.28 | 5.44 | 12.15 | 27.95 | 3.19 | 6.57 |
| 17.09 | 5.19 | 56.63 | 28.49 | 3.13 | 6.01 |
| 17.40 | 5.10 | 26.72 | 29.05 | 3.07 | 8.11 |
| 17.96 | 4.94 | 4.30 | 30.18 | 2.96 | 2.99 |
| 18.80 | 4.72 | 18.42 | 31.32 | 2.86 | 7.19 |
| 19.66 | 4.52 | 43.00 | 32.65 | 2.74 | 2.26 |
| 19.90 | 4.46 | 7.01 | 33.67 | 2.66 | 1.23 |
| 20.81 | 4.27 | 11.30 | 34.61 | 2.59 | 2.90 |
| 21.07 | 4.22 | 5.64 | 36.01 | 2.49 | 2.58 |
| 21.63 | 4.11 | 47.21 | 36.75 | 2.45 | 4.34 |
| 22.09 | 4.02 | 2.82 | 37.95 | 2.37 | 2.61 |
| 22.59 | 3.94 | 12.02 | | | |

### Example 10. Preparation of crystalline form 4 of compound 1

By means of anti-solvent addition (with dimethyl sulfoxide as the good solvent and methyl *tert-*butyl ether as the anti-solvent), a solid was precipitated, separated by filtration, and dried at room temperature to give a solid product crystalline form 4 of compound 1 as confirmed by XRPD.

### Example 11. Preparation of crystalline form 5 of compound 1

In a N₂ atmosphere, 15 mg of crystalline form 4 prepared in Example 9 was heated to 250 °C, cooled to 30 °C, and exposed to air to give crystalline form 5 of compound 1. The XRPD results are shown in FIG. 15 and Table 6. The TGA/DSC results are shown in FIG. 16. The TGA results showed a weight loss of 3.4% at 150 °C. The DSC results showed one endothermic peak at 275.8 °C.

**Table 6. Crystalline form 5**

| Pos. [°2•] | d [Å] | Rel. Int. [%] | Pos. [°2•] | d [Å] | Rel. Int. [%] |
|---|---|---|---|---|---|
| 6.90 | 12.82 | 17.77 | 23.42 | 3.80 | 64.10 |
| 9.46 | 9.35 | 7.48 | 24.33 | 3.66 | 33.39 |
| 11.36 | 7.79 | 15.19 | 24.80 | 3.59 | 28.25 |
| 11.98 | 7.39 | 91.53 | 25.32 | 3.52 | 44.09 |
| 12.33 | 7.18 | 78.76 | 26.04 | 3.42 | 41.95 |
| 13.62 | 6.50 | 63.86 | 26.76 | 3.33 | 27.24 |
| 14.86 | 5.96 | 100.00 | 28.18 | 3.17 | 22.82 |
| 16.00 | 5.54 | 78.74 | 28.99 | 3.08 | 20.93 |
| 17.10 | 5.19 | 21.90 | 30.02 | 2.98 | 17.22 |
| 17.88 | 4.96 | 64.21 | 30.64 | 2.92 | 18.94 |
| 19.05 | 4.66 | 47.44 | 31.93 | 2.80 | 15.27 |
| 19.71 | 4.50 | 36.83 | 32.78 | 2.73 | 16.53 |
| 20.67 | 4.30 | 30.11 | 35.52 | 2.53 | 9.02 |
| 21.91 | 4.06 | 41.54 | | | |

### Example 12. Preparation of crystalline form 6 of compound 1

15 mg of compound 1 prepared in Preparative Example 1 was suspended in 0.5 mL of ethanol and the mixture was stirred in a temperature cycle (50 °C to 5 °C). The solid was dried at room temperature in vacuum to give crystalline form 6 of compound 1. The XRPD results are shown in FIG. 17 and Table 7. The TGA/DSC results are shown in FIG. 18. The TGA results showed a weight loss of 8.8% at 150 °C. The DSC results showed three endothermic peaks at 104.0 °C, 279.1 °C, and 293.3 °C and one exothermic peak at 213.1 °C. The ¹H NMR results, as shown in FIG. 19, indicated no solvent residue.

**Table 7. Crystalline form 6**

| Pos. [°2•] | d [Å] | Rel. Int. [%] | Pos. [°2•] | d [Å] | Rel. Int. [%] |
|---|---|---|---|---|---|
| 5.94 | 14.89 | 44.71 | 24.59 | 3.62 | 4.43 |
| 6.97 | 12.68 | 8.19 | 25.01 | 3.56 | 12.57 |
| 11.80 | 7.50 | 100.00 | 25.20 | 3.53 | 12.15 |
| 12.70 | 6.97 | 60.80 | 25.55 | 3.49 | 7.43 |
| 13.79 | 6.42 | 20.24 | 25.89 | 3.44 | 15.16 |
| 15.31 | 5.79 | 6.35 | 26.46 | 3.37 | 15.63 |
| 16.00 | 5.54 | 22.25 | 27.33 | 3.26 | 1.94 |
| 16.23 | 5.46 | 10.92 | 28.17 | 3.17 | 5.48 |
| 17.02 | 5.21 | 44.83 | 28.58 | 3.12 | 11.16 |
| 17.25 | 5.14 | 48.86 | 29.02 | 3.08 | 4.73 |
| 18.86 | 4.71 | 14.00 | 29.50 | 3.03 | 1.96 |
| 19.09 | 4.65 | 11.50 | 30.08 | 2.97 | 4.38 |
| 19.45 | 4.56 | 37.02 | 30.90 | 2.89 | 4.27 |
| 20.07 | 4.42 | 9.83 | 31.79 | 2.81 | 2.07 |
| 20.40 | 4.35 | 11.05 | 33.68 | 2.66 | 1.40 |
| 20.92 | 4.25 | 25.82 | 34.36 | 2.61 | 2.51 |
| 22.64 | 3.93 | 12.79 | 36.06 | 2.49 | 6.32 |
| 23.16 | 3.84 | 15.31 | 36.63 | 2.45 | 2.52 |
| 23.99 | 3.71 | 29.59 | 37.12 | 2.42 | 2.88 |

### Example 13. Preparation of crystalline form 6 of compound 1

15 mg of compound 1 prepared in Preparative Example 1 was suspended in 0.5 mL of acetone/water (1:4, v/v) and the mixture was stirred in a temperature cycle (50 °C to 5 °C). The solid was dried at room temperature in vacuum to give a solid product crystalline form 6 of compound 1 as confirmed by XRPD.

### Example 14. Preparation of crystalline form 6 of compound 1

15 mg of compound 1 prepared in Preparative Example 1 was suspended in 0.5 mL of tetrahydrofuran/methyl *tert*-butyl ether (1:4, v/v) and the mixture was stirred in a temperature cycle (50 °C to 5 °C). The solid was dried at room temperature in vacuum to give a solid product crystalline form 6 of compound 1 as confirmed by XRPD.

### Example 15. Preparation of crystalline form 6 of compound 1

15 mg of compound 1 prepared in Preparative Example 1 was suspended in 0.5 mL of anisole/n-propanol (1:4, v/v) and the mixture was stirred in a temperature cycle (50 °C to 5 °C). The solid was dried at room temperature in vacuum to give a solid product crystalline form 6 of compound 1 as confirmed by XRPD.

### Example 16. Preparation of crystalline form 6 of compound 1

15 mg of compound 1 prepared in Preparative Example 1 was suspended in 0.5 mL of n-butanol and the mixture was stirred for 4 days at 50 °C. The obtained solid was dried at room temperature to give a solid product crystalline form 6 of compound 1 as confirmed by XRPD.

### Example 17. Preparation of crystalline form 6 of compound 1

15 mg of compound 1 prepared in Preparative Example 1 was subjected to gas-solid diffusion at room temperature in a methanol atmosphere for 10 days to give a solid product crystalline form 6 of compound 1 as confirmed by XRPD.

### Example 18. Preparation of crystalline form 6 of compound 1

15 mg of compound 1 prepared in Preparative Example 1 was subjected to gas-solid diffusion at room temperature in an acetone atmosphere for 10 days to give a solid product crystalline form 6 of compound 1 as confirmed by XRPD.

### Example 19. Preparation of crystalline form 6 of compound 1

15 mg of compound 1 prepared in Preparative Example 1 was subjected to gas-solid diffusion at room temperature in a tetrahydrofuran atmosphere for 10 days to give a solid product crystalline form 6 of compound 1 as confirmed by XRPD.

### Example 20. Preparation of crystalline form 6 of compound 1

15 mg of compound 1 prepared in Preparative Example 1 was subjected to gas-solid diffusion at room temperature in a 1,4-dioxane atmosphere for 10 days to give a solid product crystalline form 6 of compound 1 as confirmed by XRPD.

### Example 21. Preparation of crystalline form 6 of compound 1

15 mg of compound 1 prepared in Preparative Example 1 was subjected to gas-solid diffusion at room temperature in a cyclopentyl methyl ether (CPME) atmosphere for 10 days to give a solid product crystalline form 6 of compound 1 as confirmed by XRPD.

### Example 22. Preparation of crystalline form 6 of compound 1

15 mg of compound 1 prepared in Preparative Example 1 was subjected to gas-solid diffusion at room temperature in an ethanol atmosphere for 10 days to give a solid product crystalline form 6 of compound 1 as confirmed by XRPD.

### Example 23. Preparation of crystalline form 6 of compound 1

By means of anti-solvent addition (with methanol as the good solvent and n-heptane as the anti-solvent), a solid was precipitated, separated by filtration, and dried at room temperature to give a solid product crystalline form 6 of compound 1 as confirmed by XRPD.

### Example 24. Preparation of crystalline form 6 of compound 1

By means of anti-solvent addition (with methanol as the good solvent and water as the anti-solvent), a solid was precipitated, separated by filtration, and dried at room temperature to give a solid product crystalline form 6 of compound 1 as confirmed by XRPD.

### Example 25. Preparation of crystalline form 6 of compound 1

By means of anti-solvent addition (with acetone as the good solvent and water as the anti-solvent), a solid was precipitated, separated by filtration, and dried at room temperature to give a solid product crystalline form 6 of compound 1 as confirmed by XRPD.

### Example 26. Preparation of crystalline form 6 of compound 1

15 mg of compound 1 prepared in Preparative Example 1 was dissolved in 1.0 mL of methanol and let stand at room temperature to slowly evaporate the solvent to give a solid product crystalline form 6 of compound 1 as confirmed by XRPD.

### Example 27. Preparation of crystalline form 6 of compound 1

15 mg of compound 1 prepared in Preparative Example 1 was dissolved in 1.0 mL of acetone and let stand at room temperature to slowly evaporate the solvent to give a solid product crystalline form 6 of compound 1 as confirmed by XRPD.

### Example 28. Preparation of crystalline form 6 of compound 1

15 mg of compound 1 prepared in Preparative Example 1 was suspended in 0.5 mL of ethanol and the mixture was stirred for 4 days at room temperature. The obtained solid was dried at room temperature to give a solid product crystalline form 6 of compound 1 as confirmed by XRPD.

### Example 29. Preparation of crystalline form 6 of compound 1

15 mg of compound 1 prepared in Preparative Example 1 was suspended in 0.5 mL of cyclopentyl methyl ether and the mixture was stirred for 4 days at room temperature. The obtained solid was dried at room temperature to give a solid product crystalline form 6 of compound 1 as confirmed by XRPD.

### Example 30. Preparation of crystalline form 6 of compound 1

15 mg of compound 1 prepared in Preparative Example 1 was suspended in 0.5 mL of isopropyl acetate/n-propanol (1:4, v/v) and the mixture was stirred for 4 days at room temperature. The obtained solid was dried at room temperature to give a solid product crystalline form 6 of compound 1 as confirmed by XRPD.

### Example 31. Preparation of crystalline form 6 of compound 1

15 mg of compound 1 prepared in Preparative Example 1 was suspended in 0.5 mL of tetrahydrofuran/water (1:4, v/v) and the mixture was stirred for 4 days at room temperature. The obtained solid was dried at room temperature to give a solid product crystalline form 6 of compound 1 as confirmed by XRPD.

### Example 32. Preparation of crystalline form 6 of compound 1

15 mg of compound 1 prepared in Preparative Example 1 was suspended in 0.5 mL of methyl isobutyl ketone/*n*-hexane (1:4, v/v) and the mixture was stirred for 4 days at room temperature. The obtained solid was dried at room temperature to give a solid product crystalline form 6 of compound 1 as confirmed by XRPD.

### Example 33. Preparation of crystalline form 6 of compound 1

15 mg of compound 1 prepared in Preparative Example 1 was suspended in 0.5 mL of methanol/methyl *tert*-butyl ether (1:4, v/v) and the mixture was stirred for 4 days at room temperature. The obtained solid was dried at room temperature to give a solid product crystalline form 6 of compound 1 as confirmed by XRPD.

### Example 34. Preparation of crystalline form 6 of compound 1

15 mg of compound 1 prepared in Preparative Example 1 was suspended in 0.5 mL of 2-methyltetrahydrofuran/*n*-heptane (1:4, v/v) and the mixture was stirred for 4 days at room temperature. The obtained solid was dried at room temperature to give a solid product crystalline form 6 of compound 1 as confirmed by XRPD.

### Example 35. Preparation of crystalline form 6 of compound 1

15 mg of compound 1 prepared in Preparative Example 1 was suspended in 0.5 mL of acetone/water (1:4, v/v) and the mixture was stirred for 4 days at room temperature. The obtained solid was dried at room temperature to give a solid product crystalline form 6 of compound 1 as confirmed by XRPD.

### Example 36. Preparation of crystalline form 6 of compound 1

15 mg of compound 1 prepared in Preparative Example 1 was suspended in 0.5 mL of anisole/cumene (1:4, v/v) and the mixture was stirred for 4 days at room temperature. The obtained solid was dried at room temperature to give a solid product crystalline form 6 of compound 1 as confirmed by XRPD.

### Example 37. Preparation of crystalline form 6 of compound 1

15 mg of compound 1 prepared in Preparative Example 1 was suspended in 0.5 mL of dichloromethane/methyl *tert*-butyl ether (1:4, v/v) and the mixture was stirred for 4 days at room temperature. The obtained solid was dried at room temperature to give a solid product crystalline form 6 of compound 1 as confirmed by XRPD.

### Example 38. Preparation of crystalline form 7 of compound 1

In a N₂ atmosphere, crystalline form 6 prepared in Example 12 was heated to 150 °C to give crystalline form 7 of compound 1. The XRPD results are shown in FIG. 20 and Table 8.

**Table 8. Crystalline form 7**

| Pos. [°2•] | d [Å] | Rel. Int. [%] | Pos. [°2•] | d [Å] | Rel. Int. [%] |
|---|---|---|---|---|---|
| 6.33 | 13.96 | 48.87 | 22.12 | 4.02 | 17.30 |
| 6.69 | 13.20 | 7.38 | 22.53 | 3.95 | 13.71 |
| 8.89 | 9.95 | 5.50 | 23.01 | 3.86 | 31.66 |
| 11.38 | 7.77 | 19.14 | 23.44 | 3.79 | 14.70 |
| 11.60 | 7.63 | 64.63 | 24.04 | 3.70 | 23.24 |
| 12.77 | 6.93 | 22.42 | 24.60 | 3.62 | 18.02 |
| 13.01 | 6.81 | 77.68 | 25.01 | 3.56 | 15.89 |
| 13.51 | 6.56 | 100.00 | 25.23 | 3.53 | 17.80 |
| 15.41 | 5.75 | 11.90 | 26.34 | 3.38 | 12.43 |
| 15.92 | 5.57 | 14.50 | 26.82 | 3.32 | 11.64 |
| 16.54 | 5.36 | 38.38 | 27.32 | 3.26 | 15.68 |
| 17.38 | 5.10 | 44.05 | 27.60 | 3.23 | 13.93 |
| 18.12 | 4.90 | 72.21 | 28.05 | 3.18 | 13.09 |
| 19.00 | 4.67 | 21.16 | 28.50 | 3.13 | 9.94 |
| 20.20 | 4.40 | 45.51 | 28.89 | 3.09 | 10.49 |
| 20.99 | 4.23 | 13.67 | 29.23 | 3.06 | 9.50 |
| 21.62 | 4.11 | 13.53 | | | |

### Example 39. Preparation of crystalline form 5 of compound 1

In a N₂ atmosphere, crystalline form 7 prepared in Example 38 was heated to 220 °C, cooled to 30 °C, and exposed to air to give a solid product crystalline form 5 of compound 1 as confirmed by XRPD.

### Example 40. Preparation of crystalline form 8 of compound 1

By means of anti-solvent addition (with dimethylformamide as the good solvent and water as the anti-solvent), a solid was precipitated, separated by filtration, and dried at room temperature to give crystalline form 8 of compound 1. The XRPD results are shown in FIG. 21 and Table 9. The TGA/DSC results are shown in FIG. 22. The TGA results showed a weight loss of 14.1% at 150 °C. The DSC results showed three endothermic peaks at 87.2 °C and 290.5 °C and two exothermic peaks at 208.2 °C and 240.6 °C. The ¹H NMR results, as shown in FIG. 23, indicated about 0.7 wt% of dimethylformamide residue (much lower than the weight loss in TGA).

**Table 9. Crystalline form 8**

| Pos. [°2•] | d [Å] | Rel. Int. [%] | Pos. [°2•] | d [Å] | Rel. Int. [%] |
|---|---|---|---|---|---|
| 3.64 | 24.24 | 50.37 | 21.12 | 4.21 | 10.59 |
| 7.32 | 12.08 | 25.48 | 21.97 | 4.05 | 21.69 |
| 9.39 | 9.42 | 19.77 | 22.43 | 3.96 | 24.81 |
| 9.73 | 9.09 | 9.20 | 22.93 | 3.88 | 13.62 |
| 10.74 | 8.24 | 51.59 | 23.84 | 3.73 | 22.08 |
| 11.36 | 7.79 | 34.62 | 24.40 | 3.65 | 42.60 |
| 13.04 | 6.79 | 37.84 | 24.83 | 3.59 | 14.45 |
| 14.06 | 6.30 | 100.00 | 25.58 | 3.48 | 18.65 |
| 15.46 | 5.73 | 23.09 | 26.04 | 3.42 | 25.08 |
| 15.89 | 5.58 | 31.31 | 26.36 | 3.38 | 22.81 |
| 17.14 | 5.17 | 18.23 | 27.20 | 3.28 | 13.43 |
| 17.80 | 4.98 | 18.38 | 27.52 | 3.24 | 12.75 |
| 18.32 | 4.84 | 65.57 | 28.70 | 3.11 | 13.92 |
| 18.93 | 4.69 | 57.64 | 29.59 | 3.02 | 12.85 |
| 19.60 | 4.53 | 23.53 | 31.07 | 2.88 | 7.37 |
| 19.97 | 4.45 | 33.46 | 32.31 | 2.77 | 5.48 |
| 20.50 | 4.33 | 36.61 | 33.68 | 2.66 | 2.37 |

### Example 41. Preparation of crystalline form 3 of compound 1

Crystalline form 8 prepared in Example 40 was heated to 250 °C and cooled to room temperature to give a solid product crystalline form 3 of compound 1 as confirmed by XRPD.

### Example 42. Preparation of crystalline form 9 of compound 1

15 mg of compound 1 prepared in Preparative Example 1 was suspended in 0.5 mL of water and the mixture was stirred in a temperature cycle (50 °C to 5 °C). The solid was dried at room temperature in vacuum to give crystalline form 9 of compound 1. The XRPD results are shown in FIG. 24 and Table 10. The TGA/DSC results are shown in FIG. 25. The TGA results showed a weight loss of 3.4% at 50 °C and of 6.5% from 50 °C to 200 °C. The DSC results showed five endothermic peaks at 84.3 °C, 119.8 °C, 194.7 °C, 214.4 °C, and 289.7 °C. The ¹H NMR results are shown in FIG. 26.

The heating test results for crystalline form 9 showed that after the crystalline form 9 sample was heated to 100 °C and exposed to air, the XRPD pattern of the resultant sample was identical to that before heating.

**Table 10. Crystalline form 9**

| Pos. [°2•] | d [Å] | Rel. Int. [%] | Pos. [°2•] | d [Å] | Rel. Int. [%] |
|---|---|---|---|---|---|
| 3.36 | 26.33 | 72.40 | 20.24 | 4.39 | 20.35 |
| 7.98 | 11.08 | 38.05 | 21.51 | 4.13 | 14.22 |
| 8.86 | 9.98 | 23.02 | 22.25 | 3.99 | 58.70 |
| 9.90 | 8.93 | 76.41 | 23.96 | 3.71 | 10.55 |
| 11.05 | 8.00 | 12.23 | 26.33 | 3.38 | 23.47 |
| 13.86 | 6.39 | 23.29 | 27.25 | 3.27 | 48.40 |
| 14.66 | 6.04 | 100.00 | 28.55 | 3.13 | 15.56 |
| 15.48 | 5.72 | 55.22 | 30.68 | 2.91 | 17.96 |
| 16.87 | 5.26 | 77.33 | 31.56 | 2.83 | 6.15 |
| 18.81 | 4.72 | 17.50 | | | |

### Example 43. Preparation of crystalline form 9 of compound 1

15 mg of compound 1 prepared in Preparative Example 1 was suspended in 0.5 mL of water and the mixture was stirred for 4 days at room temperature. The obtained solid was dried at room temperature to give a solid product crystalline form 9 of compound 1 as confirmed by XRPD.

### Example 44. Preparation of crystalline form 10 of compound 1

In a N₂ atmosphere, crystalline form 9 prepared in Example 42 was held at 30 °C for 20 minutes to give crystalline form 10 of compound 1. The XRPD results are shown in FIG. 27 and Table 11.

**Table 11. Crystalline form 10**

| Pos. [°2•] | d [Å] | Rel. Int. [%] | Pos. [°2•] | d [Å] | Rel. Int. [%] |
|---|---|---|---|---|---|
| 6.80 | 13.00 | 9.91 | 17.01 | 5.21 | 40.48 |
| 7.97 | 11.10 | 16.32 | 17.45 | 5.08 | 55.29 |
| 9.22 | 9.60 | 10.23 | 19.21 | 4.62 | 26.06 |
| 10.03 | 8.82 | 49.66 | 20.56 | 4.32 | 20.96 |
| 12.69 | 6.98 | 16.66 | 22.14 | 4.01 | 31.49 |
| 13.80 | 6.42 | 44.68 | 24.59 | 3.62 | 17.08 |
| 14.49 | 6.11 | 48.34 | 28.32 | 3.15 | 11.50 |
| 15.42 | 5.75 | 100.00 | | | |

### Example 45. Preparation of crystalline form 3 of compound 1

In a N₂ atmosphere, crystalline form 10 prepared in Example 44 was heated to 205 °C and cooled to 30 °C to give a solid product crystalline form 3 of compound 1 as confirmed by XRPD.

### Example 46. Preparation of crystalline form 11 of compound 1

15 mg of compound 1 prepared in Preparative Example 1 was suspended in an acetonitrile/toluene (1:4, v/v) system and the mixture was stirred at room temperature to give crystalline form 11 of compound 1. The XRPD results are shown in FIG. 28 and Table 12. The TGA/DSC results are shown in FIG. 29. The TGA results showed a weight loss of 2.6% at 100 °C and of 7.9% from 100 °C to 250 °C. The DSC results showed three endothermic peaks at 61.9 °C, 221.2 °C, and 291.4 °C. The ¹H NMR results, as shown in FIG. 30, indicated 6.2 wt% of toluene (approximate to the weight loss from 100 °C to 250 °C in TGA).

The results of further identification of crystalline form 11 by variable-temperature XRPD showed that no significant crystalline form changes were observed when holding crystalline form 11 at 30 °C for 20 minutes and heating the sample to 100 °C in a N₂ atmosphere.

**Table 12. Crystalline form 11**

| Pos. [°2•] | d [Å] | Rel. Int. [%] | Pos. [°2•] | d [Å] | Rel. Int. [%] |
|---|---|---|---|---|---|
| 7.93 | 11.14 | 11.30 | 22.05 | 4.03 | 30.95 |
| 9.83 | 9.00 | 30.62 | 22.46 | 3.96 | 15.37 |
| 10.07 | 8.79 | 46.46 | 22.86 | 3.89 | 10.77 |
| 10.24 | 8.64 | 30.53 | 23.51 | 3.78 | 3.97 |
| 11.60 | 7.63 | 35.29 | 24.12 | 3.69 | 28.36 |
| 11.97 | 7.39 | 40.08 | 24.93 | 3.57 | 41.20 |
| 12.46 | 7.10 | 20.83 | 25.21 | 3.53 | 18.01 |
| 13.26 | 6.68 | 32.42 | 25.94 | 3.44 | 15.48 |
| 13.94 | 6.35 | 100.00 | 26.41 | 3.37 | 21.47 |
| 14.26 | 6.21 | 44.09 | 26.67 | 3.34 | 56.72 |
| 14.65 | 6.05 | 29.76 | 27.18 | 3.28 | 24.67 |
| 15.43 | 5.74 | 38.59 | 27.93 | 3.19 | 16.62 |
| 15.75 | 5.63 | 28.16 | 28.85 | 3.10 | 14.74 |
| 16.65 | 5.33 | 12.71 | 29.27 | 3.05 | 7.63 |
| 17.24 | 5.14 | 13.42 | 29.91 | 2.99 | 8.27 |
| 17.78 | 4.99 | 99.31 | 30.67 | 2.91 | 3.74 |
| 18.19 | 4.88 | 11.51 | 31.25 | 2.86 | 6.74 |
| 18.85 | 4.71 | 44.78 | 31.99 | 2.80 | 4.62 |
| 19.09 | 4.65 | 50.00 | 32.96 | 2.72 | 2.37 |
| 19.43 | 4.57 | 26.30 | 33.73 | 2.66 | 3.90 |
| 19.76 | 4.49 | 18.04 | 34.50 | 2.60 | 3.60 |
| 20.06 | 4.43 | 35.41 | 36.61 | 2.45 | 3.23 |
| 20.93 | 4.25 | 16.28 | 38.86 | 2.32 | 3.75 |
| 21.56 | 4.12 | 19.35 | | | |

### Example 47. Preparation of crystalline form 11 of compound 1

15 mg of compound 1 prepared in Preparative Example 1 was suspended in 0.5 mL of acetonitrile/toluene (1:4, v/v) and the mixture was stirred in a temperature cycle (50 °C to 5 °C). The solid was dried at room temperature in vacuum to give a solid product crystalline form 11 of compound 1 as confirmed by XRPD.

### Example 48. Preparation of crystalline form 3 of compound 1

In a N₂ atmosphere, crystalline form 11 prepared in Example 46 was heated to 250 °C, cooled to 30 °C, and exposed to air to give a solid product crystalline form 3 of compound 1 as confirmed by XRPD.

### Example 49. Preparation of crystalline form 12 of compound 1

15 mg of compound 1 prepared in Preparative Example 1 was dissolved in acetone, and the solution was let stand in a toluene atmosphere for gas-liquid diffusion at room temperature to precipitate a solid product crystalline form 12 of compound 1. The XRPD results are shown in FIG. 31 and Table 13. The TGA/DSC results are shown in FIG. 32. The TGA results showed a weight loss of 15.1% at 200 °C. The DSC results showed two endothermic peaks at 125.1 °C and 291.8 °C. The ¹H NMR results, as shown in FIG. 33, indicated a 6.2 wt% toluene residue.

**Table 13. Crystalline form 12**

| Pos. [°2•] | d [Å] | Rel. Int. [%] | Pos. [°2•] | d [Å] | Rel. Int. [%] |
|---|---|---|---|---|---|
| 5.77 | 15.32 | 64.65 | 18.01 | 4.92 | 5.93 |
| 6.21 | 14.22 | 100.00 | 18.92 | 4.69 | 4.78 |
| 7.71 | 11.47 | 2.19 | 19.34 | 4.59 | 5.16 |
| 9.84 | 8.99 | 1.38 | 20.19 | 4.40 | 4.00 |
| 11.75 | 7.53 | 16.10 | 21.03 | 4.22 | 2.68 |
| 12.07 | 7.33 | 23.98 | 21.44 | 4.14 | 2.24 |
| 12.43 | 7.12 | 11.02 | 22.18 | 4.01 | 2.20 |
| 12.78 | 6.93 | 4.04 | 23.01 | 3.87 | 4.51 |
| 13.23 | 6.69 | 1.83 | 23.67 | 3.76 | 5.03 |
| 14.29 | 6.20 | 4.44 | 24.86 | 3.58 | 2.07 |
| 14.55 | 6.09 | 2.85 | 25.18 | 3.54 | 3.38 |
| 14.93 | 5.93 | 4.06 | 25.86 | 3.45 | 6.00 |
| 15.62 | 5.67 | 1.50 | 26.73 | 3.33 | 3.41 |
| 16.20 | 5.47 | 2.72 | 28.87 | 3.09 | 3.11 |
| 16.89 | 5.25 | 7.54 | 30.33 | 2.95 | 2.03 |
| 17.16 | 5.17 | 3.92 | 31.54 | 2.84 | 1.55 |
| 17.78 | 4.99 | 4.91 | 32.83 | 2.73 | 1.54 |

### Example 50. Preparation of crystalline form 12 of compound 1

15 mg of compound 1 prepared in Preparative Example 1 was dissolved in 1.0 mL of ethyl acetate and let stand at room temperature to slowly evaporate the solvent to precipitate a solid product crystalline form 12 of compound 1 as confirmed by XRPD.

### Example 51. Preparation of crystalline form 12 of compound 1

15 mg of compound 1 prepared in Preparative Example 1 was subjected to gas-solid diffusion at room temperature in a methyl ethyl ketone (MEK) atmosphere for 10 days to give a solid product crystalline form 12 of compound 1 as confirmed by XRPD.

### Example 52. Preparation of crystalline form 12 of compound 1

15 mg of compound 1 prepared in Preparative Example 1 was dissolved in isopropyl acetate, and the solution was let stand in a methyl tert-butyl ether atmosphere for gas-liquid diffusion at room temperature. The obtained clear solution was then let stand at room temperature for evaporation to precipitate a solid product crystalline form 12 of compound 1 as confirmed by XRPD.

### Example 53. Preparation of crystalline form 13 of compound 1

In a N₂ atmosphere, crystalline form 12 prepared in Example 49 was held at 30 °C for 20 minutes to give crystalline form 13 of compound 1. The XRPD results are shown in FIG. 34 and Table 14.

**Table 14. Crystalline form 13**

| Pos. [°2•] | d [Å] | Rel. Int. [%] | Pos. [°2•] | d [Å] | Rel. Int. [%] |
|---|---|---|---|---|---|
| 6.16 | 14.35 | 100.00 | 19.89 | 4.46 | 19.59 |
| 9.56 | 9.25 | 2.40 | 20.62 | 4.31 | 8.21 |
| 11.94 | 7.41 | 63.06 | 21.24 | 4.18 | 7.79 |
| 12.45 | 7.11 | 23.03 | 22.24 | 4.00 | 7.98 |
| 13.08 | 6.77 | 5.81 | 23.52 | 3.78 | 18.86 |
| 14.26 | 6.21 | 8.97 | 24.24 | 3.67 | 10.10 |
| 14.85 | 5.96 | 7.76 | 25.15 | 3.54 | 10.08 |
| 15.58 | 5.69 | 10.73 | 26.79 | 3.33 | 11.69 |
| 16.97 | 5.22 | 27.64 | 27.83 | 3.21 | 9.74 |
| 18.10 | 4.90 | 27.50 | 30.24 | 2.96 | 6.98 |
| 19.43 | 4.57 | 16.61 | 35.73 | 2.51 | 3.41 |

### Example 54. Preparation of crystalline form 3 of compound 1

In a N₂ atmosphere, crystalline form 13 prepared in Example 53 was heated to 200 °C and cooled to 30 °C to give a solid product anhydrous crystalline form 3 of compound 1 as confirmed by XRPD.

### Example 55. Preparation of crystalline form 3 of compound 1

The preparation is identical to that in Example 3, except that compound 1 prepared in Preparative Example 1 was replaced by crystalline form 6 prepared in Example 12, and the obtained solid was crystalline form 3 of compound 1 as confirmed by XRPD.

### Example 56. Preparation of compound 1 maleate

Compound 1 (15 mg) prepared in Preparative Example 1 and maleic acid were added in a 1:1 molar ratio to 1 mL of ethyl acetate and the mixture was stirred at room temperature for 3 days. The obtained solid was separated at room temperature and dried in vacuum for 4 hours to give compound 1 maleate. The ¹H NMR results showed a 1.0:1 molar ratio of the ligand acid to free compound 1; the XRPD results indicated an amorphous form.

### Example 57. Preparation of compound 1 fumarate

Compound 1 (15 mg) prepared in Preparative Example 1 and fumaric acid were added in a 1:1 molar ratio to 1 mL of methanol/water (19:1, v:v) and the mixture was stirred at room temperature for 3 days. The obtained solid was separated at room temperature and dried in vacuum for 4 hours to give compound 1 fumarate. The ¹H NMR results showed a 1.0:1 molar ratio of the ligand acid to free compound 1; the XRPD results indicated an amorphous form.

### Example 58. Preparation of compound 1 citrate

Compound 1 (15 mg) prepared in Preparative Example 1 and citric acid were added in a 1:1 molar ratio to 1 mL of ethyl acetate and the mixture was stirred at room temperature for 3 days. The obtained solid was separated at room temperature and dried in vacuum for 4 hours to give compound 1 citrate. The ¹H NMR results showed a 1.5:1 molar ratio of the ligand acid to free compound 1; the XRPD results indicated an amorphous form.

### Example 59. Preparation of compound 1 p-toluenesulfonate

Compound 1 (15 mg) prepared in Preparative Example 1 and p-toluenesulfonic acid were added in a 1:1 molar ratio to 1 mL of ethyl acetate and the mixture was stirred at room temperature for 3 days. The obtained solid was separated at room temperature and dried in vacuum for 4 hours to give compound 1 p-toluenesulfonate. The ¹H NMR results showed a 1.0:1 molar ratio of the ligand acid to free compound 1; the XRPD results indicated an amorphous form.

### Example 60. Preparation of compound 1 mesylate

Compound 1 (15 mg) prepared in Preparative Example 1 and methanesulfonic acid were added in a 1:1 molar ratio to 1 mL of ethyl acetate and the mixture was stirred at room temperature for 3 days. The obtained solid was separated at room temperature and dried in vacuum for 4 hours to give compound 1 mesylate. The ¹H NMR results showed a 1.0:1 molar ratio of the ligand acid to free compound 1; the XRPD results indicated an amorphous form.

### Test Example 1. Humidity induction test

Some of the crystalline forms prepared in the above examples were investigated in a humidity induction test at room temperature. From the results, it is known that neither crystalline form 6 (Example 12) nor crystalline form 3 (Example 3) exhibited polymorphic transition after 30 days of preservation open to the atmosphere at room temperature at 22.5% RH to 84.2% RH, and neither crystalline form 8 (Example 40) nor crystalline form 9 (Example 42) exhibited polymorphic transition after 23 days of preservation open to the atmosphere at room temperature at 43.2% RH to 84.2% RH. The test conditions and results are summarized in Table 15.

**Table 15. Results of standing at various humidities at room temperature**

| Starting material | Solvent/system | Polymorphic transition or not | | |
|---|---|---|---|---|
| | | 10 days | 23 days | 30 days |
| Crystalline form 6 | 22.5% RH (saturated potassium acetate) | No | No | No |
| Crystalline form 3 | | No | No | No |
| Crystalline form 6 | 43.2% RH | No | No | No |
| Crystalline form 3 | | No | No | No |
| Crystalline form 8 | | No | No | - |
| Crystalline form 9 | (saturated potassium carbonate) | No | No | - |
| Crystalline form 6 | 60% RH (stability chamber) | No | No | No |
| Crystalline form 3 | | No | No | No |
| Crystalline form 8 | | No | No | - |
| Crystalline form 9 | | No | No | - |
| Crystalline form 6 | 84.2% RH (saturated potassium chloride) | No | No | No |
| Crystalline form 3 | | No | No | No |
| Crystalline form 8 | | No | No | - |
| Crystalline form 9 | | No | No | - |

| | | | | |
|---|---|---|---|---|
| - denotes not applicable | | | | |

### Test Example 2. Hygroscopicity

The hygroscopicity of the amorphous form sample (compound 1), crystalline form 6 (Example 12), and crystalline form 3 (Example 3) was evaluated by DVS testing at 25 °C at 0% RH to 95% RH. The DVS characterization results are shown in FIGs. 35-37. As shown in FIG. 35, a plateau can be observed in the desorption curve (cycle 1, desorption) of the crystalline form 6 sample from 30% RH to 95% RH, and the desorption curve and the re-adsorption curve do not intersect. The crystallinity of the sample was only slightly reduced after DVS testing. As shown in FIG. 36, the weight gain of the crystalline form 3 sample at 25 °C/80% RH was less than 0.9%, indicating almost no hygroscopicity, and the XRPD results show that crystalline form 3 did not change after DVS testing. As shown in FIG. 37, a plateau was observed in the desorption curve (cycle 1, desorption) of the amorphous form sample from 30% RH to 95% RH, and the desorption curve and the re-adsorption curve do not intersect.

### Test Example 3. Stability evaluation

### 3.1 Solid stability

In order to further evaluate the solid stability of the crystalline forms of the present invention, appropriate amounts of samples were for at 60 °C in a sealed condition for 24 hours and at 40 °C/75% RH open to the atmosphere for a week. Solid samples separated in different conditions were evaluated for their chemical stability such as polymorphic transition determined by XRPD, purity by HPLC, and water content by KF testing. The results for crystalline form 6 (Example 12) and crystalline form 3 (Example 3) before and after stability testing are summarized in Table 16. The results indicated no significant decrease in HPLC purity and no polymorphic transition for crystalline forms 3 and 6 in the test conditions.

**Table 16. Solid stability evaluation results for crystalline forms 6 and 3**

| Initial sample | Conditions | Time | Purity (%) | Relative to the initial (%) | Water content* (%) | Polymorphic transition or not |
|---|---|---|---|---|---|---|
| Crystalline form 6 | Initial | -- | 99.61 | -- | 9.0 | -- |
| | 60 °C/sealed | 1 day | 99.62 | 100.01 | 6.2 | No |
| | 40 °C/75% RH/open | 1 week | 99.56 | 99.95 | 8.7 | No |
| Crystalline form 3 | Initial | -- | 99.33 | -- | 0.9 | -- |
| | 60 °C/sealed | 1 day | 99.27 | 99.94 | 1.1 | No |
| | 40 °C/75% RH/open | 1 week | 99.27 | 99.94 | 1.3 | No |

| | | | | | | |
|---|---|---|---|---|---|---|
| *: qualified by KF test. | | | | | | |

Appropriate amounts of samples were weighed and let stand at 60 °C/75% RH open to the atmosphere for 7 days, 14 days, and 1 month. Samples of different standing times were evaluated for the physical stability of the crystalline forms by XRPD. The results before and after the stability test are summarized in Table 17. The results indicate the occurrence of polymorphic transition in the amorphous form, mild transition in crystalline form 6, and no transition in crystalline form 3.

**Table 17. Solid stability evaluation results for crystalline form 6, crystalline form 3, and amorphous form**

| Initial sample | Conditions | Time | Appearance | Crystalline form |
|---|---|---|---|---|
| Amorphous form sample | Initial | 0 days | Yellow solid | Amorphous form |
| | 60 °C/75% RH (open) | 7 days | Yellow solid | Amorphous form |
| | 60 °C/75% RH (open) | 14 days | Yellow solid | Mild transition, with a small amount of characteristic peaks |
| | 60 °C/75% RH (open) | 1 month | Yellow solid | Polymorphic transition |
| Crystalline form 3 | Initial | 0 days | Off-white solid | Crystalline form 3 |
| | 60 °C/75% RH (open) | 7 days | Off-white solid | Crystalline form 3 |
| | 60 °C/75% RH (open) | 14 days | Off-white solid | Crystalline form 3 |
| | 60 °C/75% RH (open) | 1 month | Off-white solid | Crystalline form 3 |
| Crystalline form 6 | Initial | 0 days | Off-white solid | Crystalline form 6 |
| | 60 °C/75% RH (open) | 7 days | Off-white solid | Crystalline form 6, with a small quantity of impurity peaks |
| | 60 °C/75% RH (open) | 14 days | Off-white solid | Crystalline form 6, with a small quantity of impurity peaks |
| | 60 °C/75% RH (open) | 1 month | Off-white solid | Crystalline form 6, with a small quantity of impurity peaks |

### 3.2 Formulation stability

In order to further evaluate the stability of the formulations prepared from the crystalline forms, appropriate amounts of the formulations were taken and let stand open to the atmosphere at 60 °C/75% RH. The physical stability and chemical stability of formulations prepared from crystalline form 3, crystalline form 6, or the amorphous form substance were evaluated by the polymorphic transition and dissolution (paddle method, 75 rpm) at different standing times. The results are summarized in Table 18. The results show that the amorphous form formulation was unstable and exhibited a decreasing dissolution rate, and after 30 days of exposure, the dissolution rate of the active ingredient was less than 75%; the formulation of crystalline form 6 exhibited mild transition, and after 30 days of standing open to the atmosphere, the dissolution rate of active ingredient was reduced but met the specifications; the formulation of crystalline form 3 was stable, and during the 30 days of standing open to the atmosphere, the dissolution rate of the active ingredient was constant.

**Table 18. Formulation stability evaluation results for crystalline form 6, crystalline form 3, and amorphous form**

| Initial sample | Conditions | Time | Crystalline form | Dissolution (45 min) |
|---|---|---|---|---|
| Amorphous form sample | Initial | 0 days | Amorphous form | 87% |
| | 60 °C/75% RH (open) | 14 days | Polymorphic transition, with characteristic peaks of some crystalline form | 80% |
| | 60 °C/75% RH (open) | 30 days | Polymorphic transition, with characteristic peaks of some crystalline form | 66% |
| Crystalline form 3 | Initial | 0 days | Crystalline form 3 | 93% |
| | 60 °C/75% RH (open) | 14 days | Crystalline form 3 | 90% |
| | 60 °C/75% RH (open) | 30 days | Crystalline form 3 | 90% |
| Crystalline form 6 | Initial | 0 days | Crystalline form 6 | 88% |
| | 60 °C/75% RH (open) | 14 days | Crystalline form 6, with a small quantity of impurity peaks | 84% |
| | 60 °C/75% RH (open) | 30 days | Crystalline form 6, with a small quantity of impurity peaks | 79% |

## Claims

1. A pharmaceutically acceptable salt of a compound, wherein the compound is compound 1 with a structure of

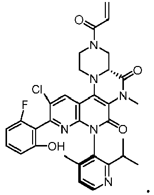

2. The pharmaceutically acceptable salt of the compound according to claim 1, wherein the pharmaceutically acceptable salt is a maleate, fumarate, citrate, p-toluenesulfonate, methanesulfonate, hydrochloride, or phosphate.

3. A polymorph, wherein the polymorph is a polymorph of a compound or a pharmaceutically acceptable salt of the compound; the compound is compound 1 with a structure of

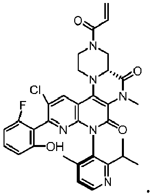

4. The polymorph according to claim 3, wherein the pharmaceutically acceptable salt is a hydrochloride or phosphate.

5. The polymorph according to claim 3, wherein,
the polymorph is crystalline form 1 of compound 1 hydrochloride having a characteristic X-ray powder diffraction peak at a 2• value selected from: 9.98±0.2°, 12.99±0.2°, 13.73±0.2°, 18.65±0.2°, 20.73±0.2°, 21.75±0.2°, 23.57±0.2°, 25.22±0.2°, and 27.27±0.2°; or
the polymorph is crystalline form 2 of compound 1 phosphate having a characteristic X-ray powder diffraction peak at a 2• value selected from: 9.74±0.2°, 10.94±0.2°, 11.55±0.2°, 13.24±0.2°, 14.20±0.2°, 15.56±0.2°, 18.40±0.2°, 19.14±0.2°, 20.54±0.2°, 24.23±0.2°, and 25.88±0.2°; or
the polymorph is crystalline form 3 of compound 1 having a characteristic X-ray powder diffraction peak at a 2• value selected from: 10.05±0.2°, 11.38±0.2°, 11.97±0.2°, 12.20±0.2°, 12.58±0.2°, 17.37±0.2°, 18.20±0.2°, 20.62±0.2°, 20.85±0.2°, and 24.95±0.2°; or
the polymorph is crystalline form 4 of compound 1 having a characteristic X-ray powder diffraction peak at a 2• value selected from: 6.00±0.2°, 6.96±0.2°, 12.11±0.2°, 13.04±0.2°, 17.09±0.2°, 17.40±0.2°, 19.66±0.2°, 21.63±0.2°, 23.24±0.2°, and 24.55±0.2°; or
the polymorph is crystalline form 5 of compound 1 having a characteristic X-ray powder diffraction peak at a 2• value selected from: 11.98±0.2°, 12.33±0.2°, 13.62±0.2°, 14.86±0.2°, 16.00±0.2°, 17.88±0.2°, 19.05±0.2°, 21.91±0.2°, 23.42±0.2°, 25.32±0.2°, and 26.04±0.2°; or
the polymorph is crystalline form 6 of compound 1 having a characteristic X-ray powder diffraction peak at a 2• value selected from: 5.94±0.2°, 11.8±0.2°, 12.7±0.2°, 17.02±0.2°, 17.25±0.2°, 19.45±0.2°, and 23.99±0.2°; or
the polymorph is crystalline form 7 of compound 1 having a characteristic X-ray powder diffraction peak at a 2• value selected from: 6.33±0.2°, 11.60±0.2°, 13.01±0.2°, 13.51±0.2°, 16.54±0.2°, 17.38±0.2°, 18.12±0.2°, 20.20±0.2°, and 23.01±0.2°; or
the polymorph is crystalline form 8 of compound 1 having a characteristic X-ray powder diffraction peak at a 2• value selected from: 3.64±0.2°, 10.74±0.2°, 11.36±0.2°, 13.04±0.2°, 14.06±0.2°, 15.89±0.2°, 18.32±0.2°, 18.93±0.2°, 19.97±0.2°, 20.50±0.2°, and 24.40±0.2°; or
the polymorph is crystalline form 9 of compound 1 having a characteristic X-ray powder diffraction peak at a 2• value selected from: 3.36±0.2°, 7.98±0.2°, 9.90±0.2°, 14.66±0.2°, 15.48±0.2°, 16.87±0.2°, 22.25±0.2°, and 27.25±0.2°; or
the polymorph is crystalline form 10 of compound 1 having a characteristic X-ray powder diffraction peak at a 2• value selected from: 10.03±0.2°, 13.8±0.2°, 14.49±0.2°, 15.42±0.2°, 17.01±0.2°, and 17.45±0.2°; or
the polymorph is crystalline form 11 of compound 1 having a characteristic X-ray powder diffraction peak at a 2• value selected from: 10.07±0.2°, 11.60±0.2°, 11.97±0.2°, 13.94±0.2°, 14.26±0.2°, 15.43±0.2°, 17.78±0.2°, 18.85±0.2°, 19.09±0.2°, 20.06±0.2°, 24.93±0.2°, and 26.67±0.2°; or
the polymorph is crystalline form 12 of compound 1 having a characteristic X-ray powder diffraction peak at a 2• value selected from: 5.77±0.2°, 6.21±0.2°, 11.75±0.2°, 12.07±0.2°, 12.43±0.2°, 12.78±0.2°, 14.29±0.2°, 14.93±0.2°, 16.89±0.2°, 17.78±0.2°, 18.01±0.2°, 18.92±0.2°, 19.34±0.2°, 20.19±0.2°, 23.01±0.2°, 23.67±0.2°, and 25.86±0.2°; or
the polymorph is crystalline form 13 of compound 1 having a characteristic X-ray powder diffraction peak at a 2• value selected from: 6.16±0.2°, 11.94±0.2°, 12.45±0.2°, 16.97±0.2°, 18.10±0.2°, 19.43±0.2°, 19.89±0.2°, and 23.52±0.2°.

6. The polymorph according to claim 5, wherein,
crystalline form 1 has a differential scanning calorimetry pattern with characteristic peaks at 62.0±5 °C and 238.0±5 °C; and/or
crystalline form 2 has a differential scanning calorimetry pattern with characteristic peaks at 68.20±5 °C, 196.0±5 °C, and 242.6±5 °C; and/or
crystalline form 3 has a differential scanning calorimetry pattern with a characteristic peak at 292.1±5 °C; and/or
crystalline form 4 has a differential scanning calorimetry pattern with characteristic peaks at 73.6±5 °C, 119.7±5 °C, 231.1±5 °C, 266.0±5 °C, and 272.8±5 °C; and/or
crystalline form 5 has a differential scanning calorimetry pattern with a characteristic peak at 275.8±5 °C; and/or
crystalline form 6 has a differential scanning calorimetry pattern with characteristic peaks at 104.0±5 °C and 279.1±5 °C; and/or
crystalline form 8 has a differential scanning calorimetry pattern with characteristic peaks at 87.2±5 °C and 290.5±5 °C; and/or
crystalline form 9 has a differential scanning calorimetry pattern with characteristic peaks at 84.3±5 °C, 119.8±5 °C, 194.7±5 °C, 214.4±5 °C, and 289.7±5 °C; and/or
crystalline form 11 has a differential scanning calorimetry pattern with characteristic peaks at 61.9±5 °C, 221.2±5 °C, and 291.4±5 °C; and/or
crystalline form 12 has a differential scanning calorimetry pattern with characteristic peaks at 125.1±5 °C and 291.8±5 °C.

7. A pharmaceutical composition, comprising: (a) the pharmaceutically acceptable salt of the compound according to claim 1 or 2 or the polymorph according to any one of claims 3-6; and (b) a pharmaceutically acceptable carrier.

8. Use of the pharmaceutically acceptable salt of the compound according to claim 1 or 2, the polymorph according to any one of claims 3-6, or the pharmaceutical composition according to claim 7 in preparing a medicament for preventing and/or treating a disease associated with KRAS G12C mutation.

9. The use according to claim 8, wherein the disease associated with KRAS G12C mutation is a cancer.

10. A method for preparing the polymorph according to claim 5, wherein
when the polymorph is crystalline form 1 of compound 1 hydrochloride, the method comprises: stirring compound 1 with hydrochloric acid in a first solvent at 10-30 °C to give crystalline form 1 of compound 1 hydrochloride; or
when the polymorph is crystalline form 2 of compound 1 phosphate, the method comprises: stirring compound 1 with phosphoric acid in a second solvent at 10-30 °C to give crystalline form 2 of compound 1 phosphate; or
when the polymorph is crystalline form 3 of compound 1, the method comprises: suspending and stirring compound 1 or crystalline form 6 of compound 1 in a third solvent at 50±10 °C to give crystalline form 3 of compound 1; or
when the polymorph is crystalline form 3 of compound 1, the method comprises: dissolving compound 1 in a first good solvent and adding a first anti-solvent while stirring to give crystalline form 3 of compound 1; or
when the polymorph is crystalline form 3 of compound 1, the method comprises: heating crystalline form 8, crystalline form 10, crystalline form 11, or crystalline form 13 of compound 1 to 200-250 °C, and cooling to 10-30 °C to give crystalline form 3 of compound 1; or
when the polymorph is crystalline form 4 of compound 1, the method comprises: subjecting compound 1 to gas-solid diffusion in a fourth solvent atmosphere at 10-30 °C to give crystalline form 4 of compound 1; or
when the polymorph is crystalline form 4 of compound 1, the method comprises: dissolving compound 1 in a second good solvent and adding a second anti-solvent while stirring to give crystalline form 4 of compound 1; or
when the polymorph is crystalline form 5 of compound 1, the method comprises: in a nitrogen atmosphere, heating crystalline form 4 or crystalline form 7 of compound 1 to 200-300 °C and cooling to 10-30 °C to give crystalline form 5 of compound 1; or
when the polymorph is crystalline form 6 of compound 1, the method comprises: stirring compound 1 in a fifth solvent in a temperature cycle to give crystalline form 6 of compound 1, the temperature cycle comprising stirring at 50 °C, cooling to 5 °C and stirring, and heating to 50 °C and stirring; or
when the polymorph is crystalline form 6 of compound 1, the method comprises: suspending and stirring compound 1 in a sixth solvent at 10-50 °C to give crystalline form 6 of compound 1; or when the polymorph is crystalline form 6 of compound 1, the method comprises: subjecting compound 1 to gas-solid diffusion in a seventh solvent atmosphere at 10-30 °C to give crystalline form 6 of compound 1; or
when the polymorph is crystalline form 6 of compound 1, the method comprises: dissolving compound 1 in a third good solvent and adding a third anti-solvent while stirring to give crystalline form 6 of compound 1; or
when the polymorph is crystalline form 6 of compound 1, the method comprises: dissolving compound 1 in an eighth solvent at 10-30 °C and standing for evaporation to give crystalline form 6 of compound 1; or
when the polymorph is crystalline form 7 of compound 1, the method comprises: in a nitrogen atmosphere, heating crystalline form 6 of compound 1 to 150±20 °C to give crystalline form 7 of compound 1; or
when the polymorph is crystalline form 8 of compound 1, the method comprises: dissolving compound 1 in a fourth good solvent and adding a fourth anti-solvent while stirring to give crystalline form 8 of compound 1; or
when the polymorph is crystalline form 9 of compound 1, the method comprises: stirring compound 1 in a ninth solvent in a temperature cycle to give crystalline form 9 of compound 1, the temperature cycle comprising stirring at 50 °C, cooling to 5 °C and stirring, and heating to 50 °C and stirring; or
when the polymorph is crystalline form 9 of compound 1, the method comprises: suspending and stirring compound 1 in a ninth solvent at 10-30 °C to give crystalline form 9 of compound 1; or when the polymorph is crystalline form 10 of compound 1, the method comprises: incubating crystalline form 9 of compound 1 in a nitrogen atmosphere at 30±5 °C to give crystalline form 10 of compound 1; or
when the polymorph is crystalline form 11 of compound 1, the method comprises: suspending and stirring compound 1 in a tenth solvent at 10-30 °C to give crystalline form 11 of compound 1; or when the polymorph is crystalline form 11 of compound 1, the method comprises: stirring compound 1 in a tenth solvent in a temperature cycle to give crystalline form 11 of compound 1, the temperature cycle comprising stirring at 50 °C, cooling to 5 °C and stirring, and heating to 50 °C and stirring; or
when the polymorph is crystalline form 12 of compound 1, the method comprises: dissolving compound 1 in a fifth good solvent and subjecting to gas-liquid diffusion in a fifth anti-solvent atmosphere to give crystalline form 12 of compound 1; or
when the polymorph is crystalline form 12 of compound 1, the method comprises: dissolving compound 1 in an eleventh solvent and standing for evaporation to give crystalline form 12 of compound 1; or
when the polymorph is crystalline form 12 of compound 1, the method comprises: subjecting compound 1 to gas-solid diffusion in a twelfth solvent atmosphere to give crystalline form 12 of compound 1; or
when the polymorph is crystalline form 13 of compound 1, the method comprises: incubating crystalline form 12 of compound 1 in a nitrogen atmosphere at 10-30 °C to give crystalline form 13 of compound 1.
